# EUROPEAN PATENT SPECIFICATION

(11) **EP 0 723 019 B1**
(45) Date of publication and mention of the grant of the patent: **18.01.2006**
(21) Application number: 96100523.8
(22) Date of filing: 20.10.1988
(51) Int. Cl.: C12N 15/82, C12N 15/29

(54) **5'-flanking region of an Arabidopsis gene coding for a 2S albumin precursor**
5'-flankierende Region eines Genes von Arabidopsis, das für einen 2S Albumin-Prekursor kodiert
Région 5'-flanquante d'un gène d'Arabidopsis codant pour un précurseur de l'albumine 2S

(30) Priority: 20.10.1987 EP 87402348
(43) Date of publication of application: 24.07.1996
(62) Divisional of application: 88402646.9
(73) Proprietor: Bayer BioScience N.V., 9052 Gent (BE)
(72) Inventor: Vandekerckhove, Joel S., B-8021 Loppem (BE); Krebbers, Enno, Alhambra, California 91803 (US); Botterman, Johan, B-9721 Zevergem-De Pinte (BE); Leemans, Jan, B-9831 Deurle (BE)
(74) Representative: Desaix, Anne

(56) References cited:
- WO-A-87/00865
- WO-A-87/07299
- DD-A- 240 911
- BIOLOGICAL ABSTRACTS, vol. 86, no. 12, 1988 Philadelphia, PA, US; abstract no. 124569, KREBBERS, E., ET AL.: "Determination of the processing sites of an Arabidopsis 2S albumin and charcterization of the complete gene family" XP002003155 & PLANT PHYSIOLOGY, vol. 87, no. 4, 1988, pages 859-866,
- JOURNAL OF THE AMERICAN OIL CHEMISTS SOCIETY, vol. 64, no. 5, May 1987, page 633 XP002003153 KNAUF, V.C., ET AL.: "Genetic engineering of ACP levels in rapeseed"
- BIOTECHNOLOGY ADVANCES, vol. 5, 1987, pages 29-45, XP002003154 SLIGHTOM, JL., ET AL.: "Advances in the molecular biology of plant seed storage proteins"

## Description

The invention relates to a 5' flanking region of an Arabidopsis gene coding for a 3S albumin.

Using plant cells as systems for the production of determined peptides has been suggested, e.g., in PCT/US86/01599. There is no evidence in that patent that the suggested methods, whose principle lies in bringing constitutively to expression said peptide according to known techniques (EP83112985.3), permit obtaining high expression levels without disturbing the plant physiology and high yields in recovering said peptides by separating them from plant proteins. This will especially be the case when the whole plant is used as such and grown in soil.

The invention aims at providing genetically modified plant DNA and plant live material including said genetically modified DNA replicable with the cells of said plant material, which genetically modified plant DNA contains sequences encoding for said determined polypeptides whose expression is under the control of a given plant promotor which conducts said expression in at least a stage of the development of the corresponding plants. This stage of development is chosen in a way that the expression occurs in plant organs or tissue which are produced in high amounts and easily recoverable.

A further object of the invention is to take advantage of the capacity of seed storage proteins to be produced in large amounts in plants and to be expressed at a determined stage of development of said plants, particularly at the seed formation stage.

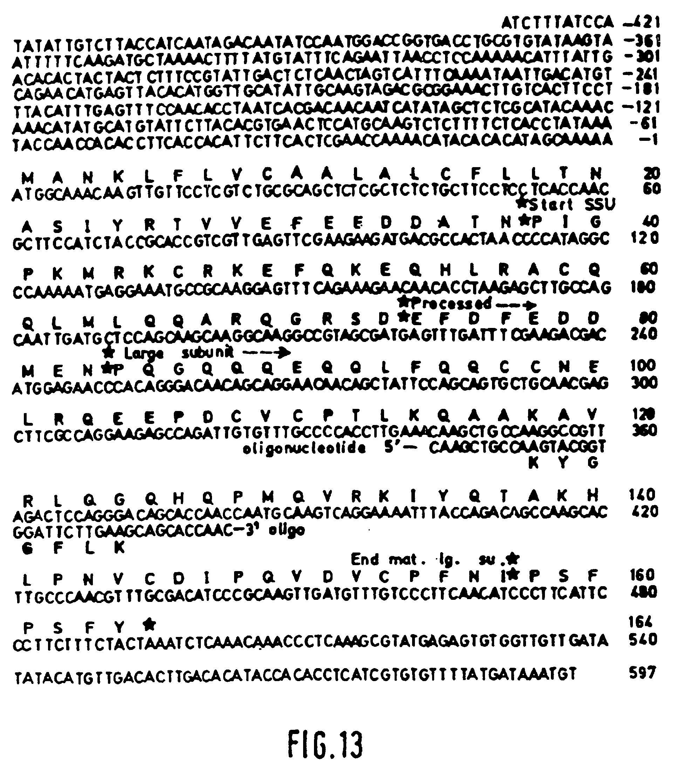

More particularly, the invention aims at providing a 5' flanking region of an Arabidopsis gene coding for a 2S albumin precursor comprising the nucleotide sequence of Figure 13 between position -431 and -1, which may be contained in plasmid pAT2S1Bg, as well as chimeric genes comprising such 5' flanking regions.

The expression of foreign genes in plants is well established (De Blaere et al., 1987). In several cases seed storage protein genes have been transferred to other plants. In several cases it was shown that within its new environment the transferred seed storage protein gene is expressed in a tissue specific and developmentally regulated manner (Beachy et al., 1985 ; Okamuro et al., 1986 ; Sengupta-Gopalan et al., 1985 ; Higgins et al., 1986). This means that the transferred gene is expressed only in the appropriate parts of the seed, and only at the normal time. It has also been shown in at least one case that foreign seed storage proteins are located in the protein bodies of the host plant (Greenwood and Chrispeels, 1985). It has further been shown that stable and functional messenger RNAs can be obtained if a cDNA, rather than a complete gene including introns, is used as the basis for the chimeric gene (Chee et al., 1986).

Seed storage proteins represent up to 90 % of total seed protein in seeds of many plants. They are used as a source of nutrition for young seedlings in the period immediately after germination. The genes encoding them are strictly regulated and are expressed in a highly tissue specific and stage specific fashion ((Walling et al., 1986; Higgins, 1984). Thus they are expressed almost exclusively in developing seed, and different classes of seed storage proteins may be expressed at different stages in the development of the seed. They are generally restricted in their intracellular location, being stored in membrane bound organelles called protein bodies or protein storage vacuoles. These organelles provide a protease-free environment, and often also contain protease inhibitors. These proteins are degraded upon flowering, and are thought to serve as a nutritive source for developing seeds. Simple purification techniques for several classes of these proteins have been described.

Seed storage proteins are generally classified on the basis of solubility and size (more specifically sedimentation rate, for instance as defined by Svedberg (in Stryer, L., Biochemestry, 2nd ed., W.H. Freeman, New York, page 599). A particular class of seed storage proteins has been studied, the 2S seed storage proteins, which are water soluble albumins and thus easily separated from other proteins. Their small size also simplifies their purification. Several 2S storage proteins have been characterised at either the protein or cDNA levels (Crouch et al., 1983 ; Sharief and Li, 1982 ; Ampe et al., 1986 ; Altenbach et al., 1987 ; Ericson et al., 1986 ; Scofield and Crouch, 1987 ; Josefsson et al., 1987 ; and work described in the present application). 2S albumins are formed in the cell from two sub-units of 6-9 and 3-4 kilodaltons (kd) respectively, which are linked by disulfide bridges.

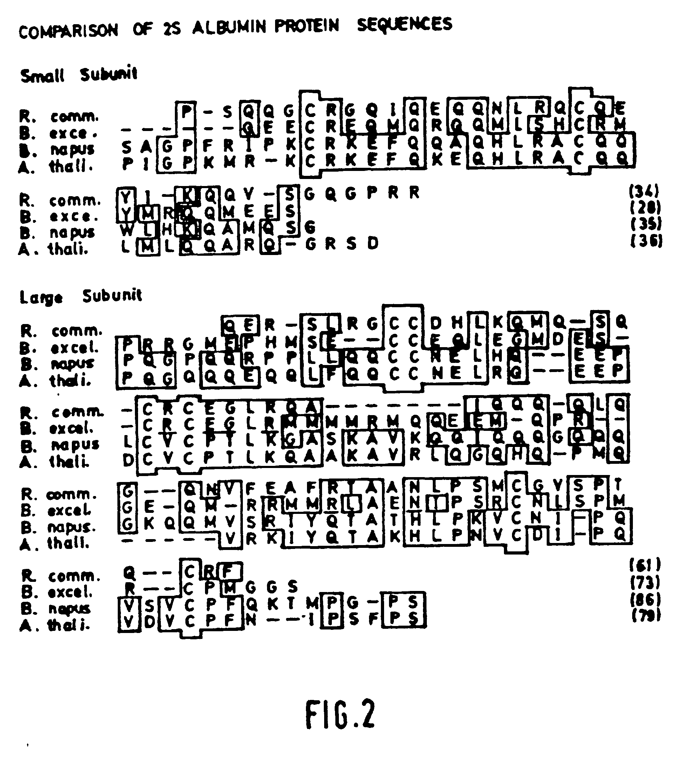

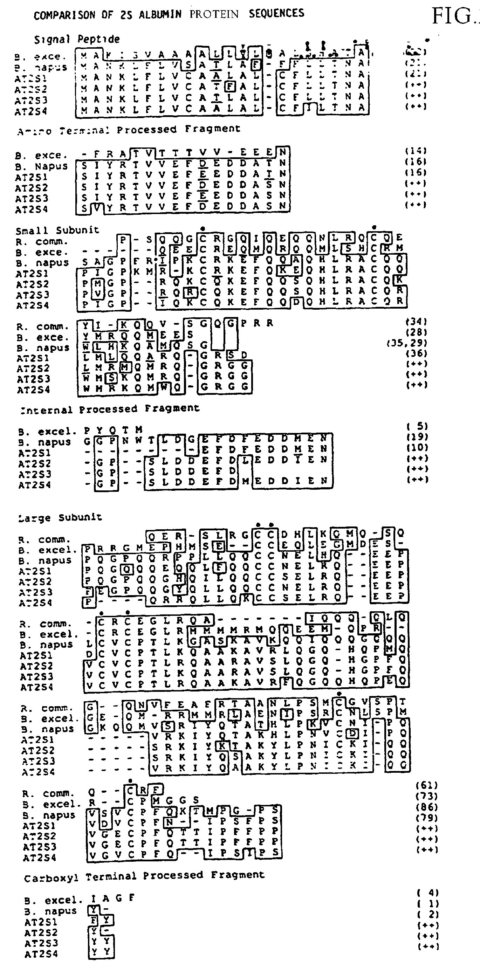

The work in the references above showed that 2S albumins are synthesized as complex prepropeptide whose organization is shared between the 2S albumins of many different species and are shown diagramatically for three of these species in figure 2. Several complete sequences are shown in figure 2.

As to fig. 2 relative to protein sequences of 2S albumins, the following observations are made. For B. napus, B. excelsia, and A. thaliana both the protein and DNA sequences have been determined.For R. communis only the protein sequence is available (B. napus from Crouch et al., 1983 and Ericson et al., 1986 ; B. excelsia from Ampe et al., 1986, de Castro et al., 1987 and Altenbach et al., 1987, R. communis from Sharief et al., 1982). Boxes indicate homologies, and raised dots the position of the cysteines.

Comparison of the protein sequences at the beginning of the precursor with standard consensus sequences for signal peptides reveals that the precursor has not one but two segments at the amino terminus which are not present in the mature protein, the first of which is a signal sequence (Perlman and Halvorson, 1983) and the second of which has been designated the amino terminal processed fragment (the so called ATPF). Signal sequences serve to ensure the cotranslational transport of the nascent polypeptide across the membrane of the endoplasmic reticulum (Blobel, 1980), and are found in many types of proteins, including all seed storage proteins examined to date (Herman et al., 1986). This is crucial for the appropriate compartmentalization of the protein. The protein is further folded in such a way that correct disulfide bridges are formed. This process is probably localized at the luminal site of the endoplasmatic reticulum membrane, where the enzyme disulfide isomerase is localized (Roden et am., 1982; Bergman and Kuehl, 1979). After translocation across the endoplasmic reticulum membrane it is thought that most storage proteins are transported via said endoplasmic reticulum to the Golgi bodies, and from the latter in small membrane bound vesicles ("dense vesicles") to the protein bodies (Chrispeels, 1983; Craig and Goodchild, 1984 ; Lord, 1985). That the signal peptide is removed cotranslationally implies that the signals directing the further transport of seed storage proteins to the protein bodies must reside in the remainder of the protein sequence present.

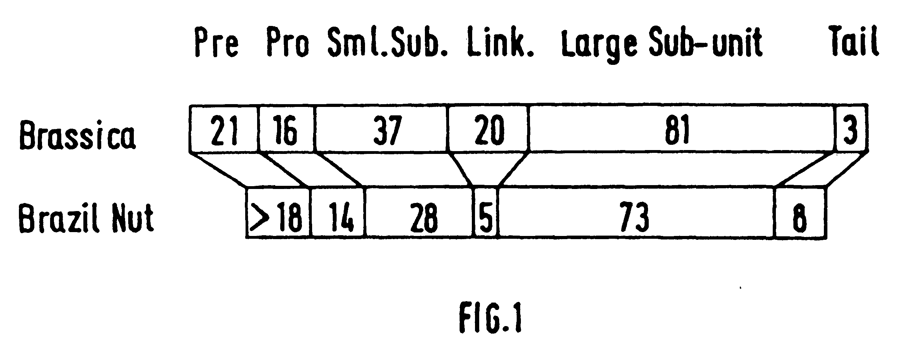

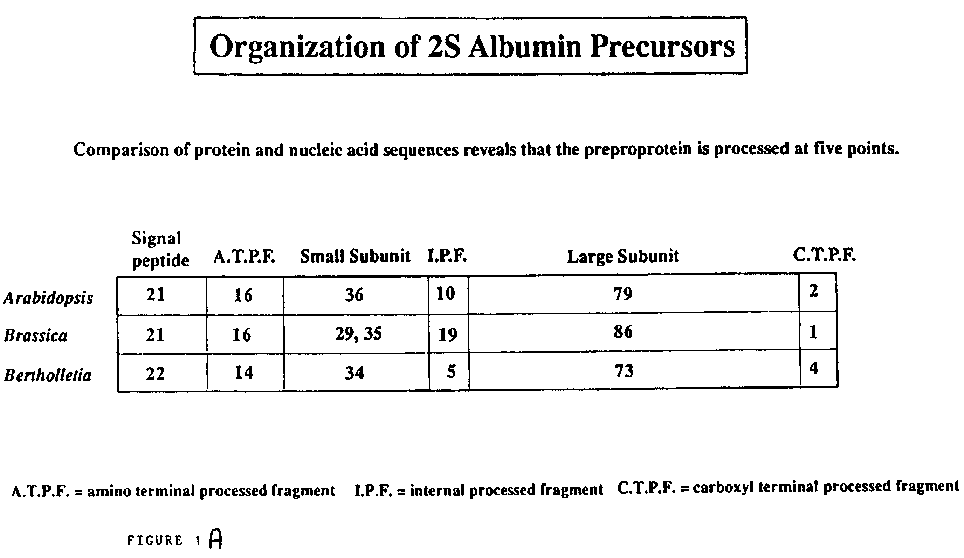

2S albumins contain sequences at the amino end of the precursor other than the signal sequence which are not present in the mature polypeptide. This is not general to all storage proteins. This amino terminal processed fragment is labeled Pro in Fig.1 and ATPF in figure 1A.

In addition, as shown in figure 1 and 1A, several amino acids located between the small and large sub-units in the precursor are removed (labeled link in Fig.1 and IPF in figure 1A, which stands for internal processed fragment). Furthermore, several residues are removed from the carboxyl end of the precursor (labeled Tail in Fig.& and CTPF in figure 1A, which stands for carboxyl terminal processed fragment). The cellular location of these latter process steps is uncertain, but is most likely the protein bodies (Chrispeels 1983 ; Lord, 1985). As a result of these processing steps the small sub-unit (Sml. Sub) and large sub-unit remain. These are linked by disulfide bridges, as discussed below.

When the protein sequences of 2S-albumins of different plants are compared strong structural similarities are observed. This is more particularly illustrated by figure 2 and 2A, which provide the aminoacid sequences of the small sub-unit and large sub-unit respectively of representative 2S storage seed albumin proteins of different plants, i.e.:
R. comm. : Ricinus communis
A. thali. : Arabidopsis thaliana
B. napus : Brassica napus
B. excel. : Bertholletia excelsa (Brazil nut)

It must be noted that in fig. 2 and 2A
- the aminoacid sequences of said sub-units extend on several lines ;
- the cysteine groups of the aminoacid sequences of the examplified storage proteins and identical aminoacids in several of said proteins have been brought into vertical alignment ; the hyphen signs which appear in some of these sequences represent absent aminoacids, in other words direct linkages between the closest aminoacids which surrounded them ;
- the aminoacid sequences which in the different proteins are substantially conserved are framed.

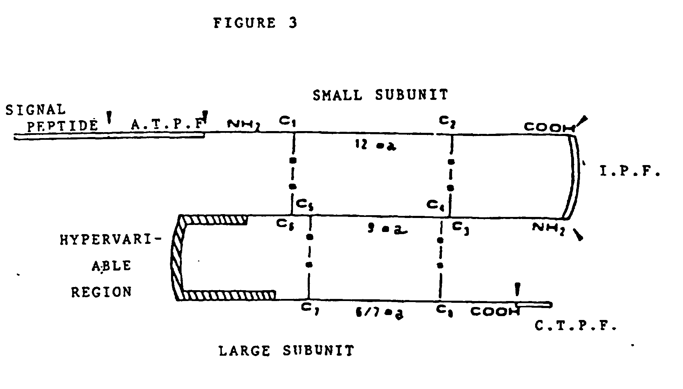

It will be observed that all the sequences contain eight cysteine residues (the first and second ones in the small sub-unit, the remainder in the large sub-unit) which can participate in disulfide bridges as diagrammatically shown in fig. 3, which represents a hypothetical model (for the purpose of the present discussion) rather than a representation of the true structure proven by experimentation of the 2S-albumin of Arabidopsis thaliana. Said hypothetical model has been inspired by the disulfide bridge mediated loop-formation of animal albumins, such as serum albumins (Brown, 1976), alpha-fetoprotein (Jagodzinski et al., 1987: Morinaga et al.; 1983) and the vitamine D binding protein where analogous constant C-C doublets and C-X-C triplets were observed (Yang et al., 1985).

Furthermore, the distances between the cysteine residues are substantially conserved within each sub-unit, with the exception of the distance between the sixth and seventh cysteine residues in the large sub-unit. This suggests that these arrangements are structurally important, but that some variation is permissible in the large sub-unit between said sixth and seventh cysteines.

The seeds of many plants contain albumins of approximately the same size as the storage proteins discussed above. However, for ease of language the term "2S albumins" will be used herein to refer to seed proteins whose genes encode a peptide precursor with the general organization shown in figure 1 and which are processed to a final form consisting of two subunits linked by disulfide bridges.

It will be appreciated that each and every cell of the cultivated plant will include the modified nucleic acid. Yet the above defined recombinant or hybrid sequence will be expressed at high levels only or mostly in the seed forming stage of the cultivated plants and, accordingly, the protein produced mostly in the seeds.

In the present invention the seed-specific promoter may originate from the same plant species or from another, subject in the last instance to the capability of the host plant's polymerases to recognize it.

Any appropriate genetic engineering technique may be used.

The general in vitro recombination techniques followed by cloning in bacteria can be used for making the chimeric genes. Site-directed mutagenesis can be used for the same purposes as further examplified hereafter. DNA recombinants, e.g. plasmids suitable for the transformation of plant cells can also be produced according to techniques disclosed in current technical literature. The same applies finally to the production of transformed plant cells in which the hybrid, can be expressed. By way of example, reference can be made to the published European applications nr. 116 718 or to International application wo 84/02913 , which disclose appropriate techniques to that effect.

The invention refers to the recombinant nucleic acids themselves particularly to the
- recombinant nucleic acids containing said seed-specific promoter,
- vectors, more particularly plant plasmids e.g., Ti-derived plasmids modified by any of the preceding recombinant nucleic acids for use in the transformation of the above plant cells.

There follows an outline of a method which can be used for the modification of 2S seed storage protein genes, their expression in transgenic plants, the purification of the 2S storage protein, and the recovery of the biologically active peptide of interest. The outline of the method given here is followed by a specific example. It will be understood from the person skilled in the art that the method can be suitably adapted for the modification of other 2S seed storage protein genes.

### 1. Replacement or supplementation of the hypervariable region of the 2S storage protein gene by the sequence of interest.

Either the cDNA or the genomic clone of the 2S albumin can be used. Comparison of the sequences of the hypervariable regions of the genes in figure 2 shows that they vary in length. Therefore if the sequence of interest is short and a 2S albumin with a relatively short hypervariable region is used, the sequence of interest can be inserted. Otherwise part of the hypervariable region is removed, to be replaced by the insert containing the segment or sequence of interest and, if appropriate, the border codons. The resulting hybrid storage protein may be longer or shorter than the non-modified natural storage protein which has been modified. In either case two standard techniques can be applied : convenient restriction sites can be exploited, or mutagenesis vectors (e.g. Stanssens et al. 1987) can be used. In both cases, care must be taken to maintain the reading frame of the message.

### 2. The altered 25 albumin coding region is placed under the control of a seed specific gene promoter.

A seed specific promoter is used in order to ensure subsequent expression in the seeds only. This facilitates recovery of the desired product and avoids possible stresses on other parts of the plant. In principle the promoter of the modified 2S albumin can be used.

In the examples below a lectin promotor from soybean and a 2S albumin promoter from Arabidopsis are used. If a chimeric gene is so constructed, a signal peptide encoding region must also be included, either from the modified gene or from the gene whose promotor is being used. The actual construction of the chimeric gene is done using standard molecular biological techniques (see example).

### 3. The chimeric gene construction is transferred into the appropriate host plant.

When the chimeric or modified gene construction is complete it is transferred in its entirety to a plant transformation vector. A wide variety of these, based on disarmed (non-oncogenic) Ti-plasmids derived from Agrobacterium tumefaciens, are available, both of the binary and cointegration forms (De Blaere et al., 1987). A vector including a selectable marker for transformation, usually antibiotic resistance, should be chosen. Similarly, the methods of plant transformation are also numerous, and are fitted to the individual plant. Most are based on either protoplast transformation (Marton et al., 1979) or transformation of a small piece of tissue from the adult plant (Horsch et al., 1985). In the example below, the vector is a binary disarmed Ti-plasmid vector, the marker is kanamycin resistance, and the leaf disc method of transformation is used.

Calli from the transformation procedure are selected on the basis of the selectable marker and regenerated to adult plants by appropriate hormone induction. This again varies with the plant species being used. Regenerated plants are then used to set up a stable line from which seeds can be harvested.

### 4. Recovery of biologically active polypeptides,

The purification of 25 plant albumins is well established (Youle and Huang, 1981; Ampe et al., 1986). It is a major protein in mature seeds and highly soluble in aqueous buffers. A typical purification of 2S-storage proteins involves the following steps : 1, homogenization of seed in dry ice and extraction with hexane ; 2, extraction with high salt buffer and dialysis against distilled water, precipitating the contaminating globulins; 3, further purification of the water soluble fraction by gel-filtration chromatography, which separates the smaller 2S-storage proteins from the larger contaminants ; and 4, final purification by ion-exchange chromatography. The exact methods used are not critical to the technique described here, and a wide range of classical techniques, including gel filtration, ion exchange and reversed phase chromatography, and affinity or immunoaffinity chromatography may be applied both to purify the chimeric 2S albumin and, after it is cleaved from the albumin, the biologically active peptide. The exact techniques used for this cleavage will be determined by the strategy decided upon at the time of the design of the flanking sequences (see above). As 2S albumins are somewhat resistant to proteases, denaturation steps should often be included before protease treatment (see example).

### 5. Assays for biologically active peptides.

Assays for the recovered product are clearly dependent on the product itself. For initial screening of plants, immunological assays can be used to detect the presence of the peptide of interest. Antibodies against the desired product will often function even while it is still part of the hybrid 2S protein. If not, it must be partially or completely liberated from the hybrid, after which peptide mixtures can be used. The screening with antibodies can be done either by classical ELISA techniques (Engvall and Pesce, 1978) or be carried out on nitrocellulose blots of proteins previously separated by polyacrylamide gel electrophoresis (Western blotting, Towbin et al., 1979). The purified peptide can be further analysed and its identity confirmed by amino acid composition and sequence analysis.

Bioassays for biological activity will of course depend upon the nature and function of the final peptide of interest.

Further characteristics of the invention will appear in the course of the non-limiting disclosure of specific examples, particularly on the basis of the drawings in which:
- Figs 1, 1A, 2A, 2 and 3 refer to overall features of 2S - storage proteins as already discussed above.
- Fig. 4 represents part of the sequence of the Brazil nut 2S-albumin obtained from the pBN2S1 plasmid obtained as indicated hereafter and related elements.
- Fig. 5 represents restriction sites used in the constructions shown in other drawings.
- Figs. 6 and 7 show diagrammatically the successive phases of the construction of a chimeric plasmid including a restriction fragment containing the nucleic acid encoding a precursor, (the herein so-called "precursor-coding nucleic acid" the whole suitable for modification by an insertion of DNA sequences encoding a polypeptide of interest, particularly through site-directed mutagenesis.
- Fig 8 shows the restriction sites and genetic map of a plasmid suitable for the performance of the above site-directed mutagenesis.
- Fig. 9 shows diagrammatically the different steps of the site-directed mutagenesis procedure of Stanssens et al (1987) as generally applicable to the modification of nucleic acid at appropriate places.
- Figs. 10, 11 and 12 illustrate diagrammatically the further steps of the modification of the abovesaid chimeric plasmid including said precursor nucleic acid to include therein, in a non essential region of its precursor nucleic acid sequence, an insert encoding a polypeptide of interest, Leu-enkephalin by way of example in the following disclosure.
- Fig. 13 represents the sequence of 1kb fragment containing the Arabidopsis thaliana 2S albumin gene and shows related elements.
- Fig. 14 provides the protein sequence of the large sub-unit of the above Arabidopsis 25 protein together with related oligonucleotide sequences.
- Fig. 15 represents the restriction map of pGSC1703.
- Fig. 16 represents the restriction map of pGSC1703A.
- Fig. 17A represents a chromatogram of an aliquot of the synthetic peptide YGGFLK, used as marker, on a C4 column. The gradient (dashed line) is isocratic at 0% solvent between 0 and 5 minutes, and solvent B increases to 100% into 70 minutes. Solvent A: 0,1% TFA in water; solvent B: 0,1% TFA in 70% CH₃CN.
- Fig. 17B represents a chromatogram of a tryptic digestion on oxidized 2S under the same conditions as done in Fig.17A. The hatched peak was collected and subjected to further purification.
- Fig. 18A represents a chromatogram of an aliquot of the synthetic peptide YGGFLK, used as a marker, on a C18 column. The gradient (dashed line) is isocratic at 0% solvent B between 0 and 5 minutes, and solvent B increases to 100% into 70 minutes. Solvent A: 0,1% TFA in water; solvent B: 0,1% TFA in 70% CH₃CN.
- Fig- 188 represents the rechromatography on the C18 column of the YGCFLK containing peak obtained from HPLC on the C4 column (see Fig. 17B). The running conditions are the same as for Fig. 18A.
- Fig. 19 represents the results of the aminoacid sequence determination on YGGFLK. The left corner box shows standard of PTH-amino acids (20 pmol each). The signal for cycles 1 to 6 is 8 times more attenuated as the reference.
- Fig. 20A represents a chromatogram showing the YGGFL peptide used as marker. This peptide is the result of a craboxypeptidase B digestion on the synthetic peptide YGGFLK. The running conditions are the same as in Fig.17A. Fig. 20B shows the isolation of the IGCFL peptide, indicated with after carboxypeptidase 8 digestion on the YGCFLK peptide, that has been isolated from the plant material.

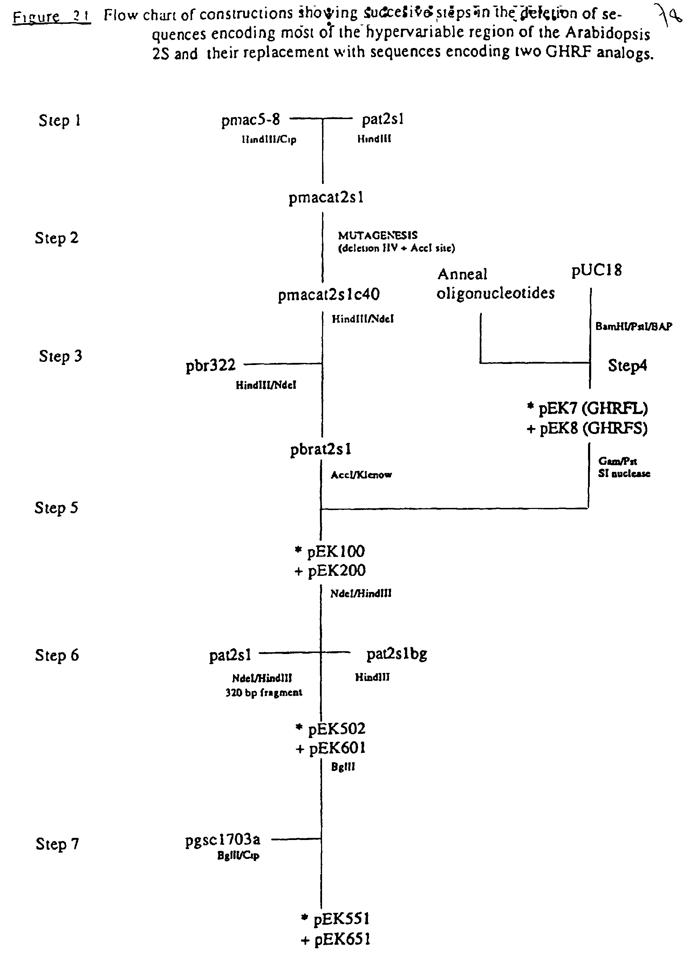

- Fig. 21 shows diagrammatically the successive phases of the construction of a chimeric 2S albumins Arabidopsis thaliania gene including the deletion of practically all parts of the hypervariable region and its replacement by a AccI site, the insertion of the sequences encoding the GHRF and cleavage sites, given by way of example in the following disclosure, in the AccI site, particularly through site-directed mutagenesis and the cloning of said chimeric gene in plant vector suitable for plant transformation.

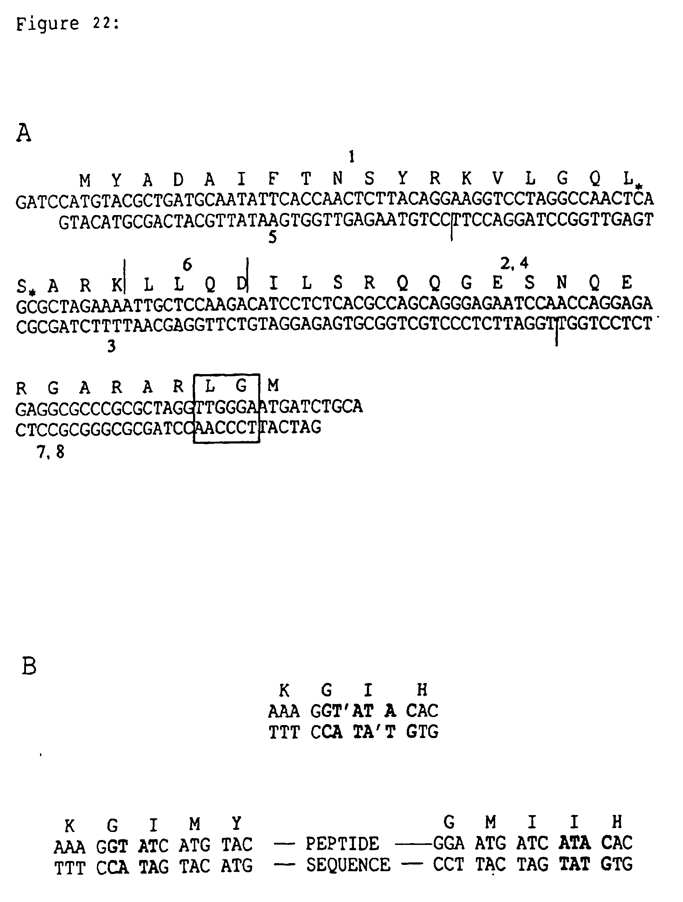

- Fig. 22A shows the eight oligonucleotides used in the constructions of the GHRFS and CHRFL genes. The limits of the oligonucleotides are indicated by vertical lines, and the numbers above and below said oligonucleotides indicate their number. In oligonucleotides 4 and 8 the bases enclosed in the box are excluded, resulting in the gene encoding GHRFs. The peptide sequence of said GHRFS and GHRFL and the methionine sequences providing the CnBr cleavage sites are shown above the DNA sequence.
- Fig. 22B shows the AccI site of the modified AT25¹ gene and the insertion of said GHRF's in said AccI site in such a way that the open reading frame is maintained.

### Example I :

As a first reference example of the method described, a procedure is given for the production of Leu-enkephalin, a pentapeptide with opiate activity in the human brain and other neural tissues (Hughes et al., 1975a). A synthetic oligomer encoding the peptide and specific protease cleavage sites is substituted for part of the hypervariable region in a cDNA clone encoding the 25 albumin of Bertholletia excelsa (Brazil nut). This chimeric gene is fused to a fragment containing the promoter and signal peptide encoding regions of the soybean lectin gene. Lectin is a 7S albumin seed storage protein (Goldberg et al., 1983). The entire construct is transferred to tobacco plants using an Agrobacterium mediated transformation system. Plants are regenerated, and after flowering the seeds are collected and the 2S albumins purified. The enkephalin peptide is cleaved from the 2S albumin using the two specific proteases whose cleavage sites are built into the oligonucleotide, and then recovered using HPLC techniques.

### 1. cDNA synthesis and screening.

Total RNA is isolated from nearly mature seeds of the Brazil nut using the method described by Harris and Dure (1981). Poly A+ RNA is then isolated using oligo dT chromatography (Maniatis et al., 1982). cDNA synthesis and cloning can be done using any of several published methods (Maniatis et al., 1902; Okayama and Berg, 1982; Land et al., 1981; Gubler and Hoffman, 1983). In the present case, the 2S albumin from Brazil nut was sequenced (Ampe et al., 1986), and an oligonucleotide based on the amino acid sequence was constructed. This was used to screen a cDNA library made using the method of Maniatis et al. (1982). The resulting clone proved to be too short, and a second library was made using the method of Gubler and Hoffman (1983) and screened using the first, shorter cDNA clone. A DNA recombinant containing the Brazil nut 2S-albumin sequence was isolated. The latter was further cloned in plasmid pUC 18. Yanisch-Perron, C., Vieira, J. and Massino, J. (1985) Gene 33, pp. 103-119.

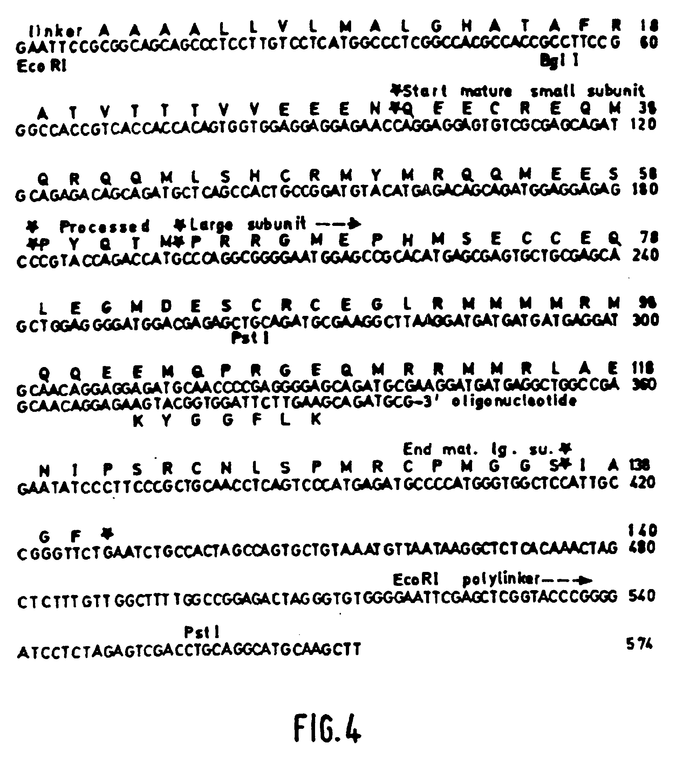

The recovered plasmid was designated pBN 251. The derived protein sequence, the DNA sequence, the region to be substituted, and the relevant restriction sites are shown in fig. 4.

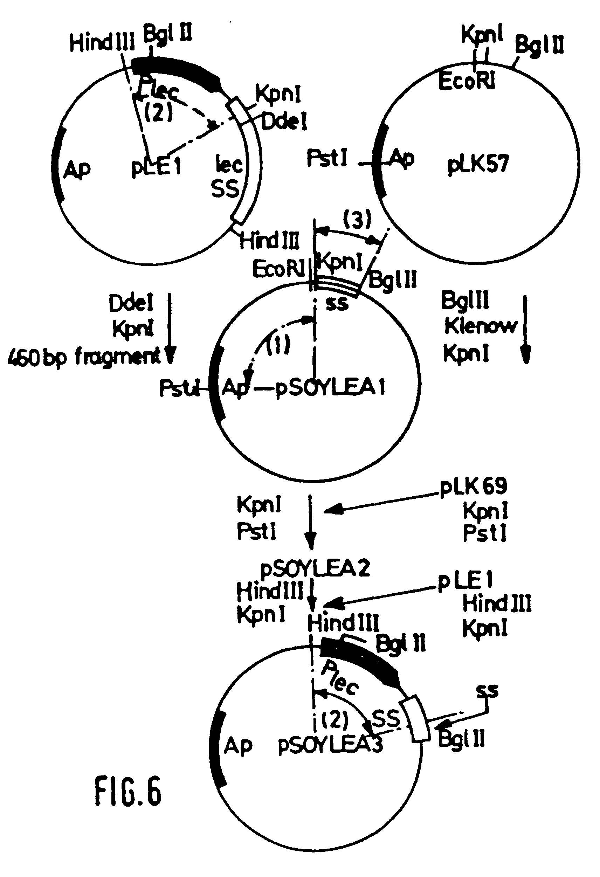

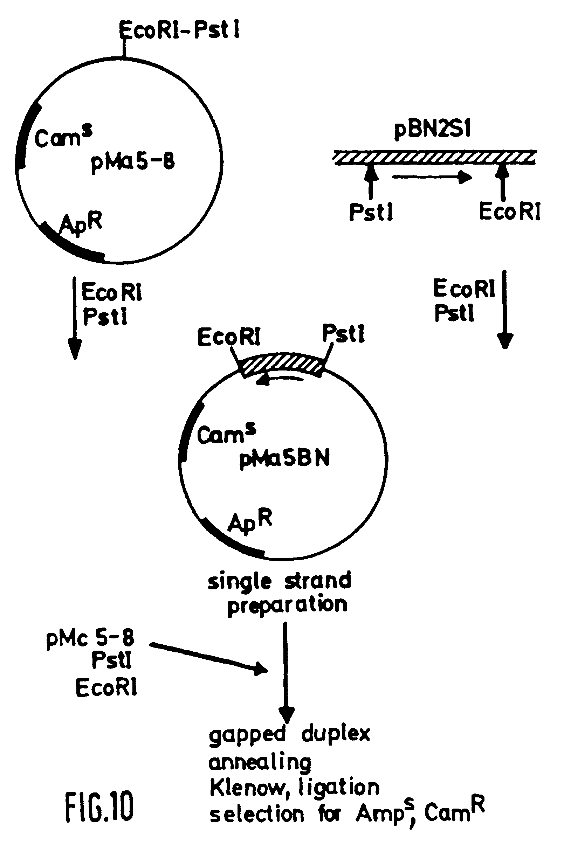

The deduced protein sequence (obtained from plasmid pBN2S1) is shown above the DNA sequence, and the proteolytic processing sites are indicated (in fig. 4). The end of the signal sequence is indicated by a Restriction sites used in the construction in figure 6, 7, 10, 11 and 12 are indicated. The polylinker of the cloning vector is shown in order to indicate the PstI site used in the latter part of the construction. The protein and DNA sequences of the peptide to be inserted are shown below the cDNA sequence, as well as the rest of the oligonucleotide to be used in the mutagenesis. During the mutagenesis procedure the oligonucleotide shown is hybridized to the opposite strand of the cDNA (see figure 10).

### 2. Construction of a chimeric gene.

The 2S albumin gene is first fused to the DNA fragment encoding the promotor and signal peptide of the soybean lectin gene. The cleavage point of the signal peptide in both lectin and Brazil nut is derived from standard consensus sequences (Perlman and Halvorson, 1983). The relevant sequences are shown hereafter as well as in figure 4.

pLe 1
*
A N S A
GCA AAC TCA GCG
CGT TTG AGT CGC
DdeI
C/TNAG

pSOYLEA 1
*
A N S D L
GCA AAC TCA GAT CTG
CGT TTC AGT CTA GAC
Bg1II
A/GATCT

pBN2S1
T A *F R A T
ACC GCC TTC CGG GCC ACC
TCC CGG AAG GCC CGG TGG
Bg1II
GCCNNNN/NGGC

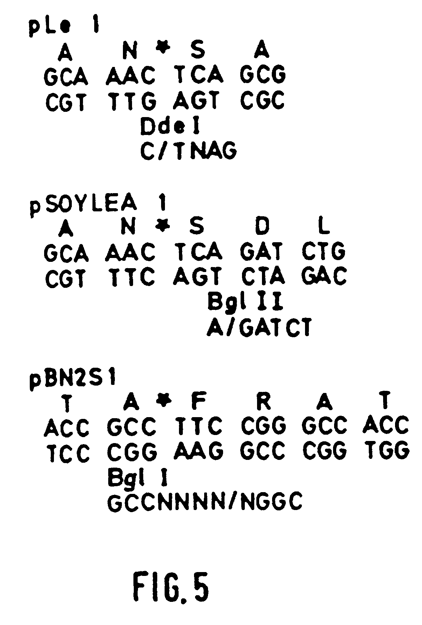

The protein and double stranded DNA sequences in the regions of the signal peptide/mature protein sequences in the plasmids pLe1, pSOYLEA and pBN2S1 are shown in figure 5. The positions and recognition sites of the restriction sites used in the constructions shown in the drawings are indicated indicates the protein cleavage site at the end of the signal sequence.

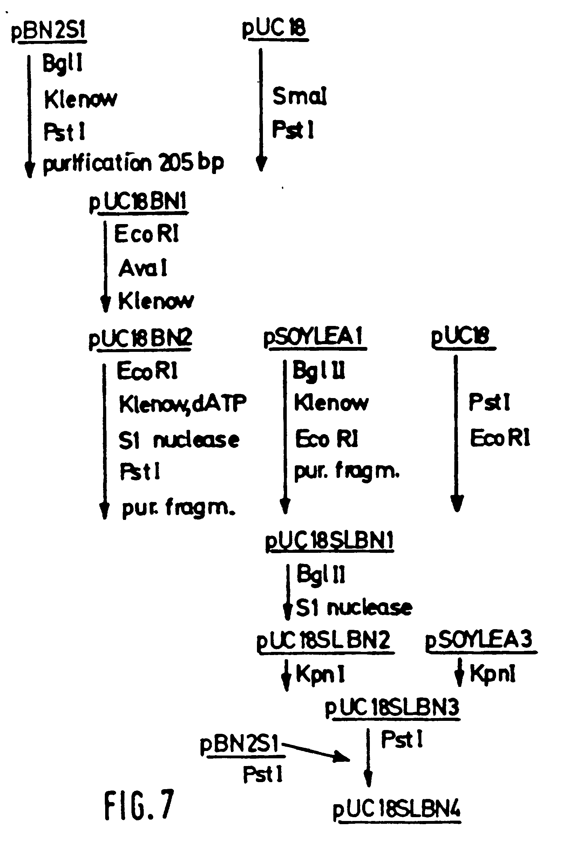

The starting point for the construction is the plasmid pLe 1 (Okamuro et al., 1987), which contains a soybean genomic HindIII fragment. This fragment includes the entire soybean lectin gene, its promoter, and sequences upstream of the promoter which may be important for seed specific expression. From this fragment a suitable soybean lectin promotor/signal sequence cassette was constructed as shown in fig. 6a. A DdeI site is present at the end of the sequence encoding the signal sequence (SS), and its cleavage site (C/TCAG) corresponds to the processing site. To obtain a useful restriction site at this processing site, a KpnI-DdeI fragment of the SS sequence (hereafter designated as "ss") is isolated from pLE1 and cloned into pLK57 (Botterman, 1986) itself linearized with KpnI and BglII. The DdeI and BglII ends are filled in with Klenow DNA Polymerase I. this reconstructs the BglII site (A/GATCT), whose cleavage site now corresponds to the signal sequence processing site (see fig. 6, 7a). The plasmid so-obtained, pSOYLEA1, thus consists of plasmid pLK57 in which the KpnI-DdeI fragment of the SS sequence (ss) initially contained in pLE1 is substituted for the initial KpnI-BglII fragment of pLK57. A HindIII site is placed in front of this fragment by substituting a KpnI-PstI fragment containing said HindIII site from pLK69 (Botterman, 1986) for the PstI-KpnI fragment designated by (1) in pSoyLea1 as shown diagrammatically in fig. 4, this intermediate construction is called pSoyLea2. In a second step the lectin promoter is reconstructed by inserting the HindIII-KpnI fragment (2) of pLE1 in pSoyLea2. As there is another BglII site present upstream of the promoter fragment, the lectin promoter/signal sequence cassette is now present as a BglII-BglII fragment in the plasmid pSoyLea3.

This cassette is now fused, in register, with a 205 bp Brazil nut cDNA fragment of plasmid pBN2S1 and containing the coding sequences for the Brazil nut pro-2S albumin (i.e., the entire precursor molecule with the exception of the signal sequence). This is done as shown in figure 5. The 205bp fragment obtained after digestion of the cDNA clone pBN2S1 (fig. 4) with BglI, treatment with Klenow DNA Polymerase I to resect the BglI protruding ends, and digestion with PstI is cloned into pUC18 (Yannish-Perron et al., 1985) which has been linearized by digestion with SmaI and PstI. The resulting plasmid, pUC18-BN1, is digested with both EcoRI and AvaI, both ends filled in, and religated. This results in the reconstruction of a new plasmid, designated pVC18-BN2, containing the desired Brazil nut coding sequence with an EcoRI site at the beginning (fig. 7).

To fuse the Brazil nut coding sequences in register to the lectin promoter/signal sequence cassette, puC18-BN2 is digested with EcoRI and the ends partially filled in using Klenow enzyme in the presence of dATP alone. The remaining overhanging nucleotides are removed with S1 nuclease, after which a PstI digest is carried out. This yields a fragment with one blunt end and one PstI digested end. The lectin promoter/signal sequence fragment is taken from pSoyLea1 (fig. 7) as an EcoRI-BglII fragment with filled in BglII ends. The two fragments are ligated together with PstI-EcoRI digested pUC18. This results in pUC18SLBN1, with a reconstructed BglII site at the junction of the signal peptide encoding sequence and the Brazil nut sequences (fig. 7) pUC18SLBN1 thus consists of the pUC18 plasmid in which there have been inserted the BglII-EcoRI fragment (shown by (3) on fig. 6) of pSoyLea1 and, upstream thereof in the direction of transcription the EcoRI-Pst-EcoRI fragment supplied by pUC18BNZ and containing the 205 bp cDNA coding sequence for the Brazil nut pro-25 albumin.

However, the reading frame is not properly maintained. In order to correct this, the plasmid is linearized with BgIII, treated with S1 nuclease, and religated. This intermediate is designated pUC1BSLBN2. The construction is finally completed in two steps by inserting the KpnI fragment carrying the 5' part of the promoter from pSoyLea3, yielding pUC18SLBN3, and inserting into the latter the PstI fragment containing the 3' part of the Brazil nut cDNA from pBN2S1. The resulting final construction, pUCSLBN4, contains the lectin promoter/signal sequence - Brazil nut cDNA sequence fusion contained within a BamHI fragment.

### 3. Substitution of part of the hypervariable region with sequences encoding enkephalin and protease cleavage sites.

The Leu-enkephalin peptide has the sequence Tyr-Gly-Gly-Phe-Leu (Hughes et al., 1975b). In order to be able to recover the intact polypeptide from the hybrid 2S albumin after purification, codons encoding Lysine are placed on either side of the enkephalin coding sequences. This allows the subsequent cleavage of the enkephalin polypeptide from the 2S albumin with the endopeptidases endolysin-C and carboxypeptidase '8 in the downstream processing steps. Finally, in order for the oligonucleotide to be capable of hybridizing to the gapped duplex molecule during mutagenesis (see below), extra sequences complementary to the Brazil nut sequences to be retained are included. The exact sequence of the oligonucleotide, determined after the study of codon usage in several plant storage protein genes, is

5'-GCAACAGGAGAAGTACGGTGGATTCTTGAAGCAGATGCC-3'

The substitution of part of the sequence encoding the hypervariable region of the Brazil nut 2S albumin is done using site-directed mutagenesis with the oligonucleotide as primer (figs. 4 and 10). The system of Stanssens et al. (1987) is used.

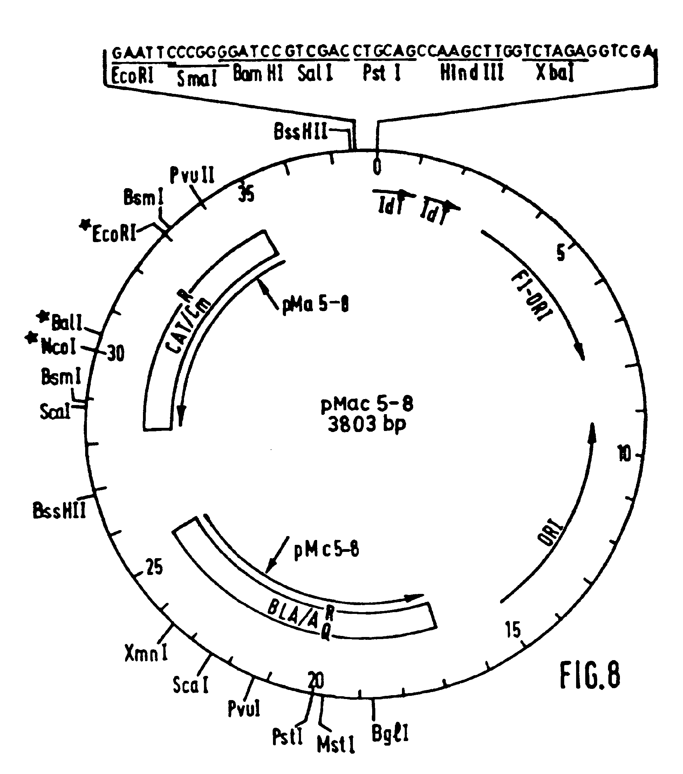

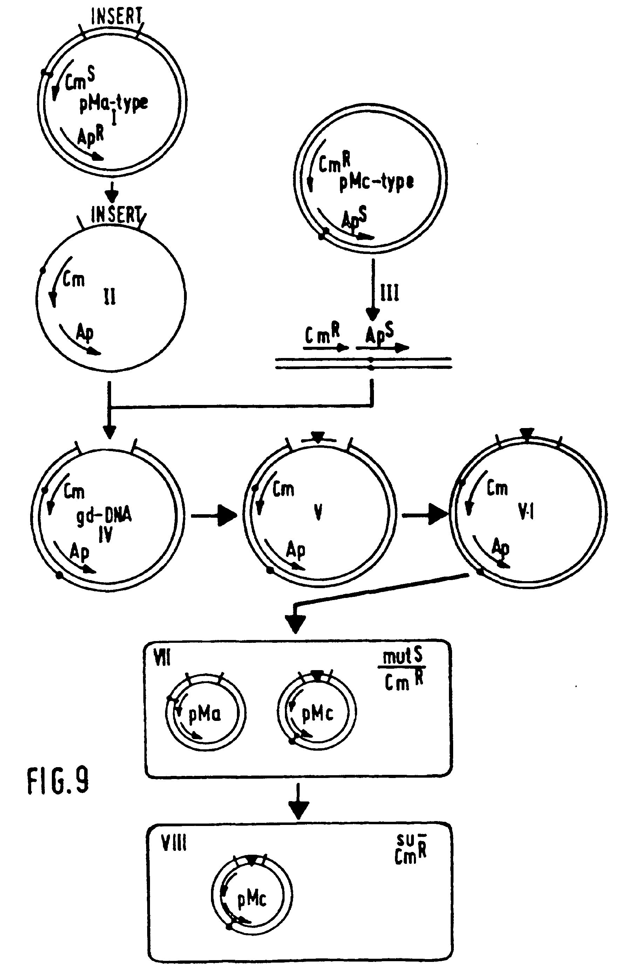

The Stanssens et al method is illustrated in fig. 9 and recalled hereinafter. It makes use of plasmid pMac5-8 whose restriction and genetic map is shown in fig. 8 and whose main features are also recalled hereinafter.

The positions of the relevant genetic loci of pMac5-8 are indicated in fig. 8. The arrows denote their functional orientation- fdT: central transcription terminator of phage fd; F1-ORI: origin of replication of filamentous phage f1; ORI: ColE1-type origin of replication; BLA/Ap^{R}: region coding for β-lactamase; CAT/Cm^{R} : region coding for chloramphenicol acetyl transferase. The positions of the amber mutations present in pMc5-8 (the bla-am gene does not contain the ScaI site) and pMc5-8 (cat-am; the mutation eliminates the unique PvuII site) are indicated. Suppression of the cat amber mutation in both supE and supF hosts results in resistance to at least 25 µg/ml Cm. pMc5-8 confers resistance to ±20 µg/ml and 100 µg/ml Ap upon amber-suppression in supE and supF strains respectively. The EcoRI, Ball and NcoI sites present in the wild-type cat gene (indicated with an asterisk) have been removed using mutagenesis techniques.

The principle of the Stanssens method as also applied to the substitution of the Leu-enkephalin peptide for the selected hypervariable region of 2S-albumin region here examplified, as described hereafter, is also first recalled hereafter:
Essentially the mutagenesis round used for the above mentioned substitution is ran as follows. Reference is made to fig. 9, in which the amber mutations in the Ap and Cm selectable markers are shown by closed circles. The symbol 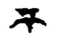 represents the mutagenic oligonucleotide. The mutation itself is indicated by an arrowhead.

The individual steps of the process are as follows:
- Cloning of the target DNA fragment into pMa5-8 (I). This vector carries on amber mutation in the Cm^{R} gene and specifies resistance to ampicillin.
- Preparation of single stranded DNA of this recombinant (II) from pseudoviral particles.
- Preparation of a restriction fragment from the complementary pMc-type plasmid (III). pMc-type vectors contain the wild-type Cm^{R} gene while an amber mutation is incorporated in the Ap resistance marker.
- Construction of gap duplex DNA (hereinafter called gdDNA) gdDNA (IV) by in vitro DNA/DNA hybridization. In the gdDNA the target sequences are exposed as single stranded DNA. Preparative purification of the gdDNA from the other components of the hybridization mixture is not necessary.
- Annealing of the synthetic oligonucleotide to the gdDNA (V).
- Filling in the remaining gaps and sealing of the nicks by a simultaneous in vitro DNA polymerase/DNA ligase reaction (VI).
- Transformation of a muts host, i.e., a strain deficient in mismatch repair, selecting for Cm resistance. This results in production of a mixed plasmid progeny (VII).
- Elimination of progeny deriving from the template strand (pMa-type) by retransformation of a host unable to suppress amber mutations (VIII). Selection for Cm resistance results in enrichment of the progeny derived from the gapped strand, i.e., the strand into which the mutagenic oligonucleotide has been incorporated.
- Screening of the clones resulting from the retransformation for the presence of the desired mutation. In the mutagenesis experiment, depicted in figure 9, Cm resistance is used as an indirect selection for the synthetic marker. Obviously, an experiment can be set up such that the Ap selectable marker is exploited. In the latter case the single stranded template (II) and the fragment (III) are the pMc- and pMa-type, respectively. A single mutagenesis step not only results in introduction of the desired mutation but also in conversion of the plasmid from pMa-type to pMc-type or vice versa. Thus, cycling between these two configurations (involving alternate selection for resistance to ampicillin or chloramphenicol) can be used to construct multiple mutations in a target sequence in the course of consecutive mutagenesis rounds.

Reverting now to the present example relative to the substitution of part of the sequence encoding the hypervariable region of the Brazil nut 2S albumin, the Stanssens et al system is thus applied as follows:

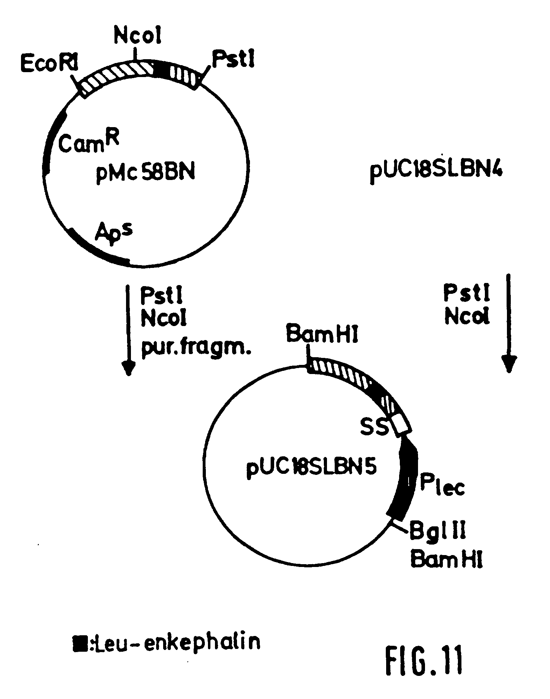

The PstI-EcoRI fragment of the chimeric gene containing the region of interest (see figs. 10, 11 and also fig.4) is inserted in a pMa vector which carries an intact beta-lactamase gene and a chloramphenicol acetyltransferase gene with an amber mutation fig. 10, so that the starting plasmid confers only ampicillin resistance but not chloramphenicol resistance. Single stranded DNA (representing the opposite strand to that shown in figure 4) is prepared and annealed with the EcoRI-PstI linearized form of a pMc type plasmid, yielding a gapped duplex molecule. The oligonucleotide is annealed to this gapped duplex. The single stranded gaps are filled with Klenow DNA polymerase I, ligated, and the mixture transformed into the appropriate host. Clones carrying the desired mutation will be ampicillin sensitive but chloramphenicol resistant. Transformants resistant to chloramphenicol are selected and analyzed by DNA sequencing. Finally, the hybrid gene fragment is inserted back into the lectin/Brazil nut chimera by replacement of the PstI-NcoI fragment in pUC18SLBN4 with the mutagenised one from pMC58BN (fig. 11). The resulting plasmid, pUC18SLBN5, contains the lectin promoter and signal sequence fused to a hybrid Brazil nut-enkephalin gene, all as a BamHI fragment.

### 4. Transformation of tobacco plants.

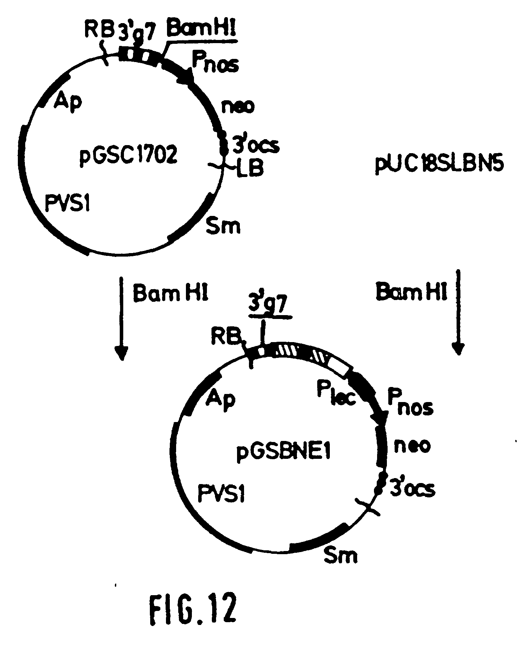

The BamHI fragment containing the chimeric gene is inserted into the BamHI site of the binary vector pGSC1702 (fig. 12). This vector contains functions for selection and stability in both E. coli and A. tumefaciens, as well as a T-DNA fragment for the transfer of foreign DNA into plant genomes (Deblaexe et al., 1987). The latter consists of the terminal repeat sequences of the octopine T-region. The BamHI site into which the fragment is cloned is situated in front of the polyadenylation signal of the T-DNA gene 7. A chimeric gene consisting of the nopaline synthase (nos) promoter, the neomycin phosphotransferase protein coding region (neo) and the 3' end of the OCS gene is present, so that transformed plants are rendered kanamycin resistant. Using standard procedures (Deblaere et al., 1987), the plasmid is transferred to the Agrobacterium strain C58C1Rif carrying the plasmid pGv2260. The latter provides in trans the vir gene functions required for successful transfer of the T-DNA region to the plant genome. This Agrobacterium is then used to transform tobacco plants of the strain SR1 using standard procedures (Deblaere et al., 1987). Calli are selected on 100 µg/ml kanamycin, and resistant calli used to regenerate plants. DNA prepared from these plants is checked for the presence of the hybrid gene by hybridization with the Brazil nut 2S albumin cDNA clone or the oligonucleotide. Positive plants are grown and processed as described below.

### 5. Purification of 2S albumins from seeds.

Positive plants are grown to seed, which takes about 15 weeks. Seeds of individual plants are harvested and homogenized in dry ice, and extracted with hexane. The remaining residue is taken up in Laemmli sample buffer, boiled, and put on an SDS polyacrylamide gel (Laemli, 1970). Separated proteins are electroblotted onto nitrocellulose sheets (Towbin et al., 1979) and assayed with a commercially available polyclonal antibody of the Leu-enkephalin antigen (UCB cat. ε i72/001, ib72/002).

Using the immunological assays above, strongly positive plants are selected. They are then grown in larger quantities and seeds harvested. A hexane powder is prepared and extracted with high salt buffer (0.5M NaCl, 0.05 M Na-phosphate pH 7.2). This extract is then dialysed against water, clarified by centrifugation (50,000xg for 30 min), and the supernatant further purified by gel filtration over a Sephadex G-75 column run in the same high salt buffer. The proteins are further purified from nonionic, non protein material ion exchange chromatogrpahy on a DEAE-Cellulose column. Fractions containing the 2S protein mixture are then combined, dialysed against 0.5 % NH₄HCO₃, and lyophilised.

### 6. Recovery of Leu-enkephalin.

The mixture of purified endogenous 2S storage proteins and hybrid 2S proteins are digested with endo-Lys-C. In order to ensure efficient proteolytic degradation, the 2S proteins are first oxidized with performic acid (Hirs, 1956). The oxidation step opens the disulfide bridges and denatures the protein. Since Leu-enkephalin does not contain amino acid residues which may react with performic acid, the opiate will not be changed by this treatment. Endo-Lys-C digested is carried out in an 0.5 % NH₄NCO₃ solution for T2 hours at 37°C and terminated by lyophilization. This digestion liberates the Leu-enkephalin, but still attached to the C terminal Lysine residue. Since the hybrid protein contains very few other lysine residues, the number of endo-Lys-C peptides is very small, simplifying further purification of the peptide. The enkephalin-Lys peptides-are purified by HPLC reversed phase chromatography using a C18 column (e.g., that commercialized under the trademard VYDAC). The gradient consists of 0.1 % trifluoroacetic acid as initial solvent (A) and 70 % acetonitrile in 0.1 % triflouroacetic acid as diluter solvent (B). A gradient of 1.5 % solvent B in A per minute is used under the conditions disclosed by Ampe et al., (1967). The purified enkephalin-Lys peptide is identified by amino acid analysis and/or by immunological techniques. It is further treated by carboxypeptidase B as disclosed by Ambler, (1972) in order to remove the carboxyl terminal Lysine residue. Finally, the separation and purification of the opiod peptide is finally achieved by reversed phase HPLC chromatography according to the method disclosed by Lewis et al., (1979).

Other methods aravailable, as illustrated in Example II.

### 7. Assay of Leu-enkephalin biological activity.

Enkephalins inhibit [³H]-naloxone binding in sodium-free homogenates of guinea pig brain. Opiod acivity can be assayed as the ability to inhibit specific [³H]-naloxone binding to rat brain membranes (Pasternak et al., 1975) as previously described (Simantov et al., 1976). One unit of opiod activity "enkephalin" was defined as that amount that yields 50% occupancy in a 200 µl assay (Colquhaun et al., 1913).

### Example II:

As a demonstration of the flexibility of the technique, a procedure for the production of Leu-enkephalin using a different 2S albumin is given. In this case, instead of using a cDNA clone from Bertholletia excelsa as basis for the construction, a genomic clone isolated from Arabisopsis thaliana is used. Since a genomic clone is used the gene's own promoter is used, simplifying the construction considerably. To further demonstrate the generality of the technique, the altered 2S albumin gene is brought to expression in three different plants: tobacco, Arabidopsis and Brassica napis, a relative of Arabidopsis which also has a 25 albumin (see introduction). Many of the details of this example are similar to the previous one and are thus described more briefly.

### 1. Cloning of the Arabidopsis thaliana 2S albumin gene.

Given the ease of purification of 2S albumin (see introduction, example 1), the most straightforward way to clone the Arabidopsis 25 albumin gene is to construct oligonucleotide probes based on the protein sequence. The protein sequence was determined by standard techniques, essentially in the same way as that of the Brazil nut 2S albumin (Ampe et al.. 1986). Figure 13 shows the sequence of the 1 kb HindIII fragment containing the Arabidopsis thaliana 2S albumin gene. The deduced protein sequence is shown above the DNA sequence, and proteolytic processing sites are indicated. The end of the signal sequence is indicated by a , and SSU indicates small subunit. The protein and DNA sequences of the peptide to be inserted are shown below the cDNA sequence, as well as the rest of the oligonucleotide to be used in the mutagenesis. During the mutagenesis procedure the oligonucleotide shown is hybridized to the opposite strand of the DNA sequence shown. The Nde I site used to check the orientation of the HindIII fragment during the construction is underlined (bp-117). The numbering system is such that the A of initiation codon is taken as base pair 1.

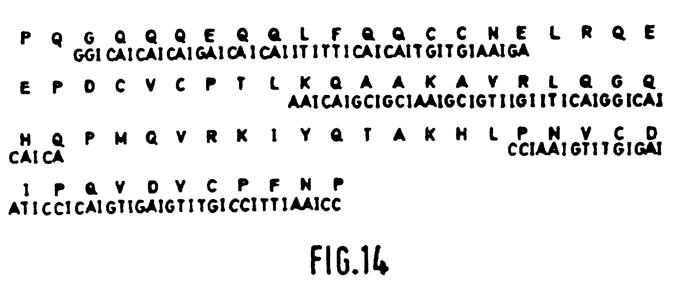

The difficulty in using oligonucleotide probes is that more than one codon can encode an amino acid, so that unambiguous determination of the DNA sequence is not possible from the protein sequence. Hence the base inosine was used at ambiguous positions. The structure of inosine is such that while it does not increase the strength of a hybridization, it does not decrease it either (Ohtsuka et al., 1985; Takahashi et al., 1985). On this basis, three oligonucleotide probes were designed as shown in figure 14. The protein sequence of the large sub-unit of the 2S albumin of Arabidopsis thaliana. Under the protein sequence are the sequences of the oligonucleotides used as hybridization probes to clone the gene. I designates Inosine.

The three oligonucleotides were used to screen a genomic library of Arabidopsis DNA constructed in the phage Charon 35 (Loenen and Blattner, 1983) using standard methods (Maniatis et al., 1982; Benton and Davis, 1977). The oligonucleotides were kinased (Miller and Barnes, 1986), and hybridizations were done in 5X SSPE (Maniatis et al., 1982), 0.1% SDS, 0.02% Ficoll, 0.02% Polyvinylpyrolidine, and 50 µg/ml sonicated herring sperm DNA at 45°C. Filters were washed in 5X SSPE, 0.1 % SDS at 45 degrees for 4-8 minutes. Using these conditions, a clone was isolated which hybridized with all three oligonucleotide probes. Appropriate regions were subcloned into pUC18 (Yanisch-Perron et al., 1985) using standard techniques (Maniatis et al., 1982) and sequenced using the methodology of Maxam and Gilbert (1980). The sequence of the region containing the gene is shown in figure 13.

### 2. Substitution of part of the hypervariable region with sequences encoding enkephalin and protease cleavage sites.

The gene isolated above was used directly for construction of a Leu-enkephalin/2S albumin chimera. As in the first example, an oligo was designed incorporating the Leu-enkephalin sequence and lysine encoding codons on either side of it, in order to be able to recover the enkephalin polypeptide in the downstream processing steps, and extra sequences complementary to the flanking Arabidopsis sequences in order for the oligonucleotide to be able to hybridize to the gapped duplex molecule during the mutagenesis. The resultant oligonucleotide has the sequence: 5'-CAAGCTGCCAAGTACGGTGGATTCTTGAAGCAGCACCAAC- 3'
its position in the sequence is shown in figure 8.

The region containing the gene and sufficient flanking regions to include all necessary regulatory signals is contained on a 3.6 kb BglII fragment, inserted in the cloning vector pJB65 (Botterman et al., 1987). The clone is called pAT2S1Bg. The region to be mutagenized is contained on 1 kb Hind III fragment within the 3.6 kb BgIII fragment, and this smaller fragment is inserted into the HindIII site of the pMa5-8 vector of Stanssens et al., (1987) (fig. 5c). The orientation is checked using an asymmetric NdeI site (figure 8). The mutagenesis is carried out using exactly the strategy described in step 3 of example 1.

Subsequently the hybrid gene is reinserted into the larger fragment with the mutagenized one using standard techniques (Maniatis et al., 1982). The orientation is again checked using the NdeI site.

### 3. Transformation of plants.

The BglII fragment containing the hybrid gene and sufficient flanking sequences both 5' and 3' to the coding region to insure that appropriate signals for gene regulation are present is inserted into the BamHI site of the same binary vector, pGSC1702, used in example 1 (figure 12). This vector is described in section 4 of example 1. Transformation of tobacco plants is done exactly as described there. The techniques for transformation of Arabidopsis thaliana and Brassica napus are such that exactly the same construction, in the same vector, can be used. After mobilization to Agrobacterium tumefaciens as described in section 4 of example 1, the procedures of Llyod et al., (1986) and Klimaszewska et al. (1985) are used for transformation of Arabidopsis and Brassica respectively. In each case, as for tobacco, calli can be selected on 100 *µ*g/ml kanamycin, and resistant calli used to regenerate plants. DNA prepared from such plants is checked for the presence of the hybrid gene by hybridization with the oligonucleotide used in the mutagenesis (In the case of tobacco and Brassica, larger portions of the hybrid construct could be used, but in the case of the Arabidopsis these would hybridize with the endogenous gene.).

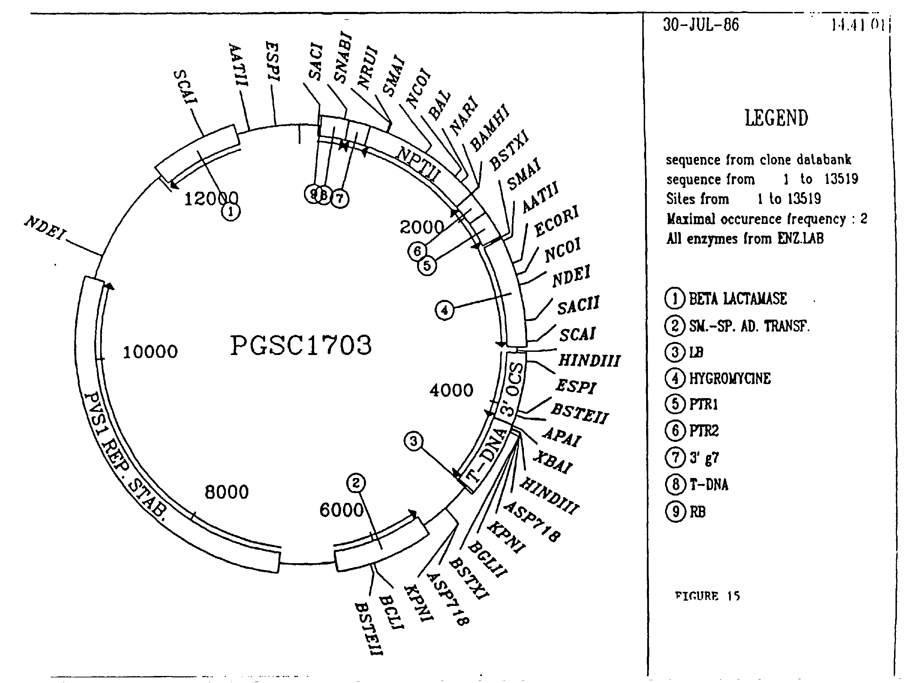

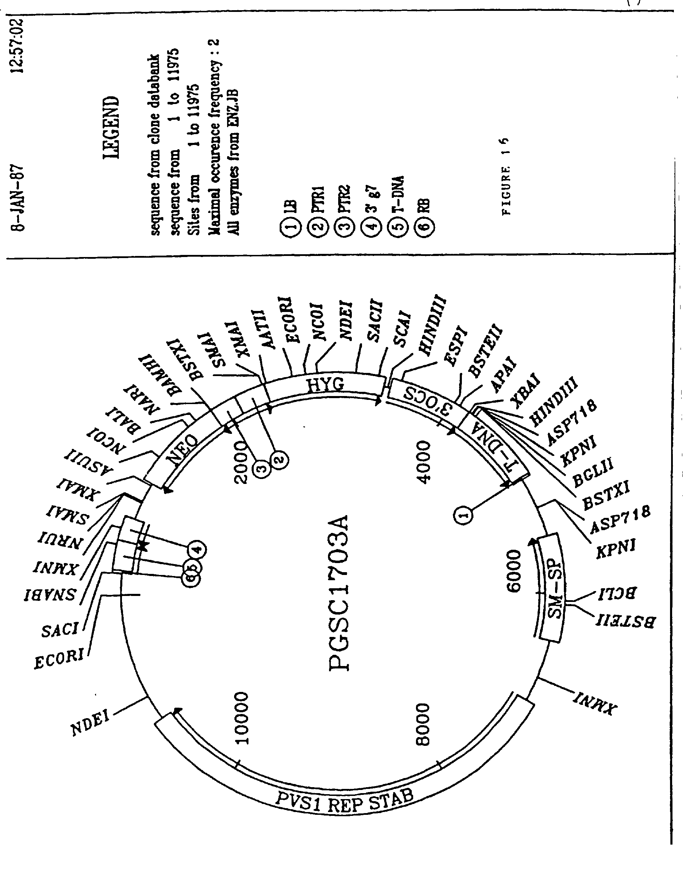

In the embodiment of the invention, BglII fragment containing the hybrid gene and sufficient flanking sequences both 5' and 3' to the coding region to insure that appropriate signals for gene regulation are present is inserted into the BglII site of the binary vectors pCSC1703 (Fig. 15) or pGSC1703A (Fig. 16). pCSC1703 contains functions for selection in both E. coli and Agrobacterium ,as well as the T-DNA fragments allowing the transfer of foreign DNA into plant genomes (Deblaere et al., 1987) It further contains the bidirectional promotor TR (Velten et al., 1984) with the neomycine phosphotransferase protein coding region (HPTII) and the 3' end of the ocs gene. It do not contain a gene encoding ampicillin resistance, as pCSC1702 does, so that carbenicillin as well as claforan can be used to kill the Agrobacteria after the infection step. Vector pGSC1703A contains the same functions as vector pGSC1703, with an additional gene encoding hygromycine transferase. This allows the selection of the transformants on both kanamycin as hygromycine. Transformation of tobacco plants is done exactly as described in section 4 of Example I, whereby the hybrid gene is inserted into the plant transformation vector pGSC1703. Transformation of Arabidopsis thaliana and Brassica napus were done with pGSC1703A in which the hybrid AT251 gene has been inserted. After mobilization to Agrobacterium tumefaciens C58C1Rif carrying the plasmid pMP90 (Koncz and Schell, 1986), which latter provides in trans and vir gene functions but which do not carry a gene encoding ampicillin resistance, the procedures of Lloyd et al., (1986) and Klimaszewska et al. (1985) are used for transformation of Arabidopsis and Brassica respectively. Carbenicillin is used to kill the Agrobacterium after co-cultivation occured. In each case, as for tobacco, calli can be selected on 100 µg/ml kanamycin, and resistant calli used to regenerate plants. DNA prepared from such plants is checked for the presence of the hybrid gene by hybridization with the oligonucleotide used in the mutagenesis. (In the case of tobacco, larger portions of the hybrid construct could be used, but in the case of Brassica and Arabidopsis these would hybridize with the endogenous gene.)

### 4. Purification of 25 albumins from seeds and further processing

positive plants from each species are grown to seed. In the case of tobacco this takes about 15 weeks, while for Arabidopsis and Brassica approximately 6 weeks and 3 months respectively are required. Use of different varieties may alter these periods. Purification of 2S albumins from seeds, recovery of the Leu-enkephalin, and assaying the latter for biological activity are done as follows.

### Methods used for the isolation of Enkephalin from Arabidopsis seeds

Two methods were used to isolate Enkephalin from Arabidopsis seeds. First, a small amount of seeds isolated from several individual transformants was screened for the presence of chimeric 2S albumins. This is done because, as described by Jones et al., (1985), expression of introduced genes may vary widely between individual transformants. Seeds from individual plants seen by this preliminary screening were then used to isolate larger amounts and determine yields more accurately. Both procedures are described below.

### A) Fast screening procedure for Enkephalin-containing 2S proteins

Seeds of individual plants (approximately 50 mg) were collected and ground in an Eppendorf tube with a small plastic grinder shaped to fit the tube. No dry ice is used in this procedure. The resulting paste was extracted three times with 1 ml of heptane and the remaining residue dried. The powder was suspended in 0.2 ml of 1M NaCl and centrifuged for 5 min in an Eppendorf centrifuge. This extraction was repeated three times and the supernatants combined, giving a total volume of approximately o.5ml. This solution was diluted 20 fold with water, giving a final NaCl concentration of 0.05M. This was stored overnight at 4°C and then spun at 5000 rpm in a Sorvall SS-34 rotor for 40 min. The resulting supernatant was passed over a disposable C18 cartridge (SEP-PAC, Millipore, Milford, Massachusetts, U.S.A.). The cartridges were loaded by injecting the 10ml supernatant with a syringe through the columns at a rate of 5 ml/min. The cartridge was then washed with 2 ml of 0.1% TFA and proteins were desorbed by a step elution with 2 ml portions of a 0.1% TFA solution containing 7%, 14%, 21% etc. up to 70% acetonitrile. The fractions eluting in the range from 28% to 49% acetonitrile are enriched for 2S albumins as judged by SDS-polyacrylamide gel analysis performed on aliquots taken from the different fractions. The 2S albumin-containing fractions were combined and dried in a Speed vac concentrator (Savant Instruments).

The combined fractions were reconstituted in 0.95 ml 0.1% TFA in water, filtered through an HV-4 Millex filter (Millipore), and applied to a reversed phase C₆ column 25 cm in length and 0.46 cm in diameter (Vydac 214TP54, pore size 300 angstrom, particle size 5 *µ*m). The HPLC equipment consisted of 2 pumps (model 510), a gradient controller (model 680) and an LC spectrophotometer detector (Lambda-Max model 481, all from waters, Milford, Massachusetts, U.S.A.). The gradients were run as follows: solution A was 0.1% TFA in H₂O, solution B 0.1% TFA in 70% CH₃CN. For 5 minutes, a solution of 0% B, 100% A was run over the column, after which the concentration of B was raised to 100% in a linear fashion over 70 minutes. The column eluate was detected by absorbance at 214 nm. The fractions containing 2S albumins were collected and dried in a Speed Vac concentrator.

In order to obtain a more complete digestion with proteases it is recommended that the proteins be denatured by oxidizing the disulfide bridges with performic acid. This is done by adding 0.5 ml of a solution made by mixing 9 ml of formic acid and 1 ml of 30% H₂O₂ at room temperature. The solution was made 2 hours before use. The reaction is allowed to proceed for 30 min at 0°C and terminated by drying in a Speed Vac concentrator. Traces of remaining performic acid were removed by twice adding 500 µl of water and lyophilizing the sample.

The residue was redissolved in 0.75 ml of 0.1M Tris-HCl pH 8.5 after which 4 µg of TPCK-treated trypsin (Worthington) was added. The reaction was placed at 37°C for 3 hours, after which it was terminated by the addition of 10 µl of TFA and stored at -20°C prior to analysis. The resulting peptide mixture is separated by HPLC using the columns and gradient mixtures described above. As a standard, a peptide of the same sequence as that expected (YGGFLK) was synthesized using standard techniques on a Biolynx 4175 peptide synthesizer (LKB). This peptide was run over the column and the retention time determined. The mixture of peptides resulting from the trypsin digest was then loaded on the same column and peptides with the same retention time as the standard were collected, dried, and reloaded on a C18-reversed phase column. The elution time of the marker peptide again served as a reference for the correct position of the enkephalin containing peptide. The identity of this peptide was confirmed by amino acid sequencing, which also allowed a rough quantitation. Four plants of the six transformants analyzed were shown to contain significant quantities of Leu-enkephalin-By way of example the detailed analysis and processing steps are given below for one of these said four plants.

### B) Larger scale isolation and processing of Enkephalin from Arabidopsis seeds

### Grinding and initial extraction

2.11 g of seeds from said plant were ground in ,a mortar in dry ice. Lipids were removed from the resulting powder by extracting three times with 5 ml of heptane. The resulting residue was dried.

### Protein extraction

The powder was dissolved in approximately 4 ml of 1.0M NaCl. The resulting paste was spun in an SS-34 rotor at 17,500 rpm for 40 min. After each spin the supernatant was transferred to a fresh tube and the pellet again resuspended in 4 ml of 1.0M NaCl. This procedure was repeated three times. The three supernatants (12 ml total) were passed through a 0.45 µm filter (HA, Millipore).

### Isolation of 2S albumins via gel filtration

The 12 ml of solution from the previous step was passed over a Sephadex G-50 medium (Pharmacia) column in two batches of 6ml. The column was 2.5 cm in diameter, 100 cm in length, and run at a flow rate of approximately 27 ml/hr in 0.5M NaCl. Fractions of approximately 7 ml were collected. The fractions were monitored for the protein in two ways. First, total protein was detected by applying 10 µl of each fraction on a piece of Whatman 3MM paper, indicating the fraction numbers with a pencil. The spots are dried for 1 min in warm air and the proteins fixed by a quick (30 sec) immersion of the paper sheet in a 10% TCA solution. The sheet is then transferred to a Commassie Blue solution similar to that used for polyacrylamide gel staining. After 1 min, the paper is removed and rinsed with tap water. Protein containing fractions show a blue spot on a white background. The minimum detection limit of the technique is about 0.05 mg/ml. Those fractions containing protein were assayed for the presence of 2S albumins by adding 2 µl of the 7 ml fraction to 10 µl of sample buffer and then loading 6 µl of this mixture on a 17.5% polyacrylamide minigel. Those fractions shown to contain 25 albumins were pooled; the total volume of the pooled fractions was 175 ml.

### Desalting of the isolated 2S albumins

This was done via HPLC over a C₄ column 25 cm in length and 0.46 cm in diameter (Vydac 214TP54, pore size 300 angstrom, particle size 5 µm). The HPLC equipment consisted of 2 pumps (model 510), a gradient controller (model 680) and an LC spectrophotometer detector (Lambda-Max model 481, all from Waters, Milford, Massachusetts, U.S.A.). 21 ml of the 175 ml were loaded on this system in 6 runs of 3.5 ml each. The gradients were run as follows: Solution A was 0.1% TFA in H₂O, solution B 0.1% TFA in 70% CH₃CH. For 5 minutes, a solution of 0% B, 100% A was run over the column, after which the concentration of B was raised to 100% in a linear fashion over 70 minutes. During each run the 2S albumin fraction was collected, and after all 6 runs these fractions pooled and divided into 3 tubes, each of which therefore contained 7/175 of the 2S albumins from the 2.11 g seeds. Each of the aliquots was processed further separately and used for quantitative estimation of yields.

### Trypsin Digest

Prior to digestion with trypsin the three aliquots were oxidized as described above. The trypsin digest was carried out essentially as described above. 0.95 ml of 0.1M Tris-HCl pH 8.5 was added to each aliquot, which was supplemented with 50 µg of trypsin (Worthington) and the reaction allowed to proceed for 4 hr at 37°C. Isolation of the YGGFLK peptide

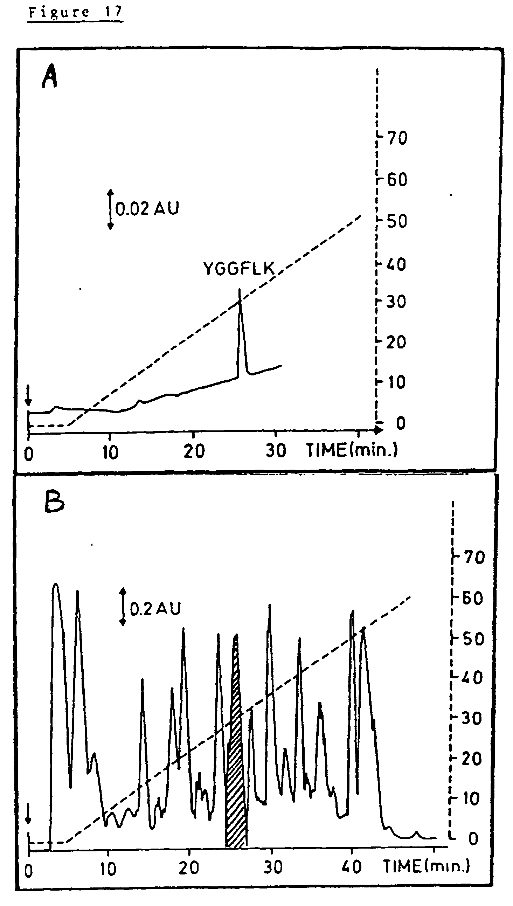

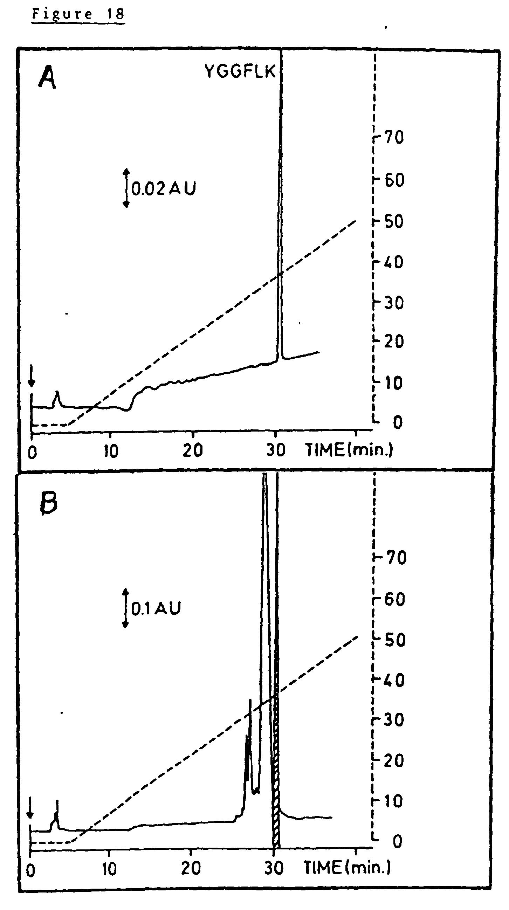

The enkephalin peptide containing the carboxyl terminal lysine residue was isolated using sequential HPLC steps. As described in the small scale isolation procedure above, a peptide of the same sequence as that expected was synthesized and run over an HPLC system using the same column and gradient conditions described in the desalting step above. The retention time of the synthetic peptide was determined (Fig. 17A). The three trypsin digests were then (separately) loaded on the same column and the material with the same retention time as that of the synthetic peptide collected (the hatched area in Fig. 17B) and dried. The same procedure was then followed using the same equipment and gradients except that a C18 column (25 × 0.46 cm, vydac 218TP104 material of pore size 300 angstrom and particle size 10 *µ*m) was used. Again material with the same retention time as the synthetic peptide was collected (Fig. 18A and 18B). This resulted in three preparations each derived from 7/175 of the total 2S albumin.

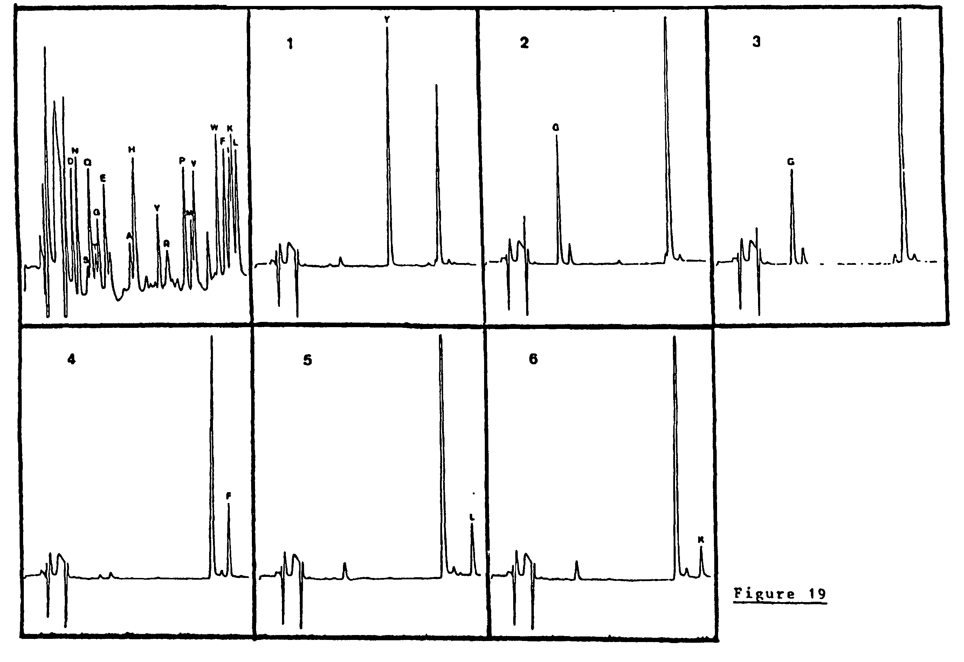

1/20 of the material in one of these three aliquots was used to check the sequence of the isolated peptide. This was determined by automated gas-phase sequencing using an Applied Biosystems Inc. (U.S.A.) 470A gas-phase sequenator. The stepwise liberated phenylthiohydantoin (PTH) amino acid derivatives were analyzed by an on-line PTH-amino acid analyzer (Applied Biosystems Inc. 120A). The sequenator and PTH-analyzer were operated according to the manufacturer's instructions. The HPLC-chromatograms of the liberated PTH-amino acids from cycles 1 through 6 are shown in figure 19. The sequence was as expected YGGFLK. The yield of PTH-amino acid of the first cycle was used for calculate the yield of this intermediate peptide (251-277 nmol/gr seed).

### Removal of the extra Lysine from the enkephalin

The three aliquots resulting from the previous step were resuspended in 100 µl of 0.2M N-Ethylmorpholine pH 8.5 (Janssen chimica, Belgium) and one third of each treated with 0.2 µg of carboxypeptidase B (Boehringer Mannheim, sequencing grade) at 37°C. The three aliquots were treated for 5, 12, and 17 minutes respectively, but all three digests proved to be equally effective. After digestion the enkephalin was purified by HPLC using the same equipment, column, and gradients as described under desalting above.

The final yield of enkephalin was determined by doing an amino acid analysis. An aliquot representing 1/150 of the total amount of the above mentioned three aliquots was hydrolyzed in 400 µl of 6N HCl, 0.05% phenol at 110°C for 24 h. The hydrolysate was dried and amino acids derivitised into phenylthiocarbamoyl (PTC) residues (Bildingmeyer et al., 1984). Three separate aliquots of the PTC residue mixture were quantified using the PICO-TAG amino acid analysis system (Waters, Millipore, Milford, Massachusetts, U.S.A.). Yields of enkephalin peptide were calculated for each of the three samples using alpha amino-butyric acid as an internal standard. Based on an average of the three determinations a final yield of 206 nmol enkephalin/g seed was calculated.

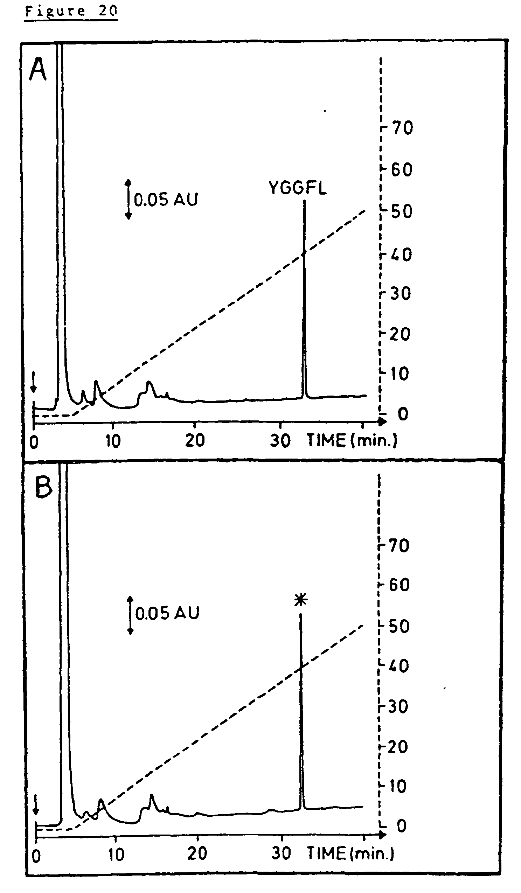

The identity of the peptide finally obtained was verified in three ways. First, its amino acid composition, which showed molar ratios of Gly, 1.76; Tyr, 1.00; Leu, 1-15 and Phe, 102. Secondly, ita retention time on a reversed phase HPLC column match that of a reference enkenephalin peptide (fig. 20) and finally its amino acid sequence was determined. These criteria unambiguously identify the peptide isolated from chimeric 2S albumins as being Leu-enkephalin.

There follows a list of bibliographic references which have been referred to in the course of the present diclosure to the extent when reference has been made to known methods for achieving some of the process steps referred to herein or to general knowledge which has been established prior to the performance of this invention.

It is further confirmed
- that plasmid pGV2260 has been deposited with the DSM on 2799 on December, 1983.
- plasmid pSOYLEA has been deposited with the DSM on 4205 on August 3, 1987; and
- plasmid pBN 261 has been deposited with the DSM on 4205 on August 3, 1987.
- plasmids pMa5-8 have been deposited with the DSM on 4567 and pMc on 4566 on May 3, 1988.
- plasmid pAT2S1 has been deposited with the DSM on 4879 on October 7, 1988
- plasmid pAT2S1Bg has been deposited with the DSM on 4878 on October 7, 1988
- plasmid pGSC1703A has been deposited with the DSM on 4880 on October 7, 1988
- plasmid pEK7 has been deposited with the DSM on 4876 on October 7, 1988.
- plasmid pEK8 has been deposited with the DSM on 4877 on October 7, 1988.

no with standing the fact that they all consist of constructs that the person skilled in the art can reproduce then from available genetic material without performing any inventive work.

### REFERENCES

Altenbach, S.B., Pearson, K.W., Leung, F.W., sun, S.S.M (1987) Plant Mol. Biol. 8, 239-250.
Aubler, R.P. (1972) Methods in Enzym. 25, 143-154. Ampe C., Van Damme, J., de castro, L.A.B., Sampaio,
M.J.A.M., van Montagu, M. and Vandekerckhove, J. (1986) Bur. J. Biochem. 159, 597-604.
Beachy, R.N., Chen, Z.-L., Horsch, R.B., Rogers, S.G., Hoffman, N.J. and Fraley, R.T. (1985) EMBO J. 4, 3047-3053.
Benton, W.D. and Davis, R.W. (1977) Science 196, 180-182.
Bergman; L.W. and Kuehl, W.N. (1979) J. Biol. Chem. 254, 5690-5694.
Bildingmeyer, B.A., Cohen S.A. and Tarvin T.L-(1984) J. of Chromatography 336, 93-104.
Blobel, (1980) Proc. Natl. Acad Sci. 77, 1496-1500. Bolivar, F., Rodriguez, R.L., Greene, P.J., Betlach, M.C., Heynecker, H.L., Boyer, H.W., Crosa, J.H. and Falkow, S. (1977) Gene 2, 95.
Botterman, J. (1986) PhD. Thesis, state University of Gent.
Brown, J.W.S., wandelt, Ch., Maier, U., Dietrich, G., Schwall, N., and Feix, G. (1986) EMBO workshop "Plant Storage Protein Genes" Program and abstract page 17, Eds. J. Brown and G. Feix, University of Freiburg, 1986. Chee, P.P., Klassy, R.C. and Slighton, J.L. (1986) Gene 41, 47-57.
Chrispeels, N.J. (1983) Planta 158, 140-152.
Colqshoun, D. (1973) in Drug Receptors, Ed. Rang, H.P. (University Park Press, Baltimore, Md.) pp. 149-182.
Craig, S. and Goodchild, D.J. (1964) Protoplasma 122, 35-44.
Crouch, M.L., Tembargo, K.M., Simon, A.E. and Perl, R. (1983) J. Mol. Appl. Gen. 2, 273-283.
De Blaere, R., Reynaerts, A., Hofte, H., Hernalsteens, J.-P., Leemans, J. and Van Montagu, M. (1987) Methods in Enzymology (in press)· (Still in press B.K. 7)
De Castro, L.A.B., Lacerada, Z., Aramayo, R.A., Sampaio, M.J.A.M. and Gander, E.S. (1987) Mol. Gen. Genet. 206, 338-343.
Dellaporta S.L.; J.; wood, J. and Hicks, B. (1983). Plant Molecular Biology Reports 1, 19-21.
Engvall, E. and Pesce, A.J. (1978) Scand. Immunol. Suppl. 7.
Ericson, M.L., Rodin, J., Lenman, M., Glimeliums, K., Lars-Goran, J. and Rak, L. (1986) J. Biol. Chem. 261, 14 576-14 581.
Goldberg, R.B., Hoachek, G., and Vodkin, L.O. (1983) Cell 33, 465-475.
Greenwood, J.S. and Chrispeels, M.J. (1985) Plant Physiol. 79, 65-71.
Gubler, U. and Hoffman, B.J. (1983) Gene 25, 263-269.
Harris, B. and Dure, L. (1981) Biochemistry 17, :3250-3256.
Herman, E.M., Shannon, L.M. and Chrispeels, M.J. (1986) In Molecular Biology of Seed Storage Proteins and Lectins, L.M. Shannon and M.J. Chrispeels Eds., American Society of Plant Physiologists.
Higgins, T.J.V. (1984) Ann. Rev. Plant Physiol. 35, 191-221.
Higgins, T.J.V., Llewellyn, D., Newbigin, E. and Spencer, D. (EK ? Symposium ref. + date). Hirs, C.H.W. (1956) J. Biol. Chem. 219, 611-621.
Horsch, R.B., *Fry*, J.E., Hoffmam, N.L., Bichholtz, D., Rogers, S.G. and Fraley, R.T. (1985) Science 227, 1229-1231.
Hughes, J., Smith, T., Morgan, B. and Fothergill, L. (1975a) Life Sci. 16, 1753-1758.
Hughes, J., Smith, T.W., Kosterlitz, H.W. Fothergill, L.A., Morgan, B.A. and Morris, H.R. (1975b) Nature 258, 577-579.
Jagodzinski, L., Sargent, T., Yang. M., Glackin, C., Bonner, J. (1987) Proc. Natl. Acad. Sci. USA. 78, 3521-3525.
Nones J.D.G.; Dunsmuir, P. and Bedbrook, J. (1985) EMBO J. 4 (10), 2411-2418.
Josefason, L-G.; Lenman, M., Ericson, M.L. and Rask, L. (1987). J. Biol. Chem. 262 (25), 12196-12201.
Klimassevska, K. and Keller, W.A. (1985). Plant cell Tissue organ Culture, 4, 183-197.
Konez, C. and Schell, J. (1986) Mol. Gen. Genet. 204, 383-396.
Land, H., Grez, M., Hauser, H., Lindenmaier, W. and Schuetz, G. (1981) Nucl. Acids. Rev. 9, 2251-2266.
Lewis, R.V., Stein, S. and Udenfriend, S. (1979) Int. J. peptide Protein Res. 13, 493-497.
Lloyd, A.M., Barnason, A.R., Rogers, S.G., Byrne, M.C., Praley, R.T. and Horch, R.B. (1986) science 234, 464-466.
Loenan and Blattner (1983) Gene 26, 171.
Lord, J.M. (1985). Bur. J. Biochem. 146, 403-409-maniatis, T., Fritsch, E.F. and Sambrook, J. (1982) Molecular Cloning. Cold Spring Harbor Laboratory, Cold Spring Harbor, New York.
Marton, L. , Wullems, G.J., Molendijk, L. and Schilperoort, R.A. (1979) Nature, 277, 129-131. Maxam, A.M. and Gilbert, W. (1950) Methods in Enzymology 65, 499-560.
Miller, J.K. and Barnes, W.M. (1986) Proc. Natl. Acad. Sci U.S.A. 83, 1026-1030.
Morinaga, T., Sakai, N., Wegmann, T., Tanaoki, T. (1983) Proc. Hatl. Acad. Sci. 80, 4604-4606.
Ohtsuka, E., Matsuki, S., Ikehara, M., Takahashi, Y. and Matsubara, K. (1985) J. Biol. Chem. 260, 2605-2608.
Okamurb, J.K., Jofuku, K.D. and Soldbery, R.B. (1986) Proc. Natl. Acad. Sci. USA. 83, 8240-8244.
Okayama, H. and Berg, P. (1992) Mol. cell. Biol. 2, 161-170.
Pasternak, G.W., Wilson, H.A. and Snyder, S.H. (1975) Mol. Pharmacol. 11, 340-350.
Perlman; D. and Halvorson, H.o. (1983) J. Mol. Biol. 167, 391-409.
Roden, L.T., Miflin, B.J., Freedman, R.B. (1982; FEBS Lett. 138, 121-124.
Scofield, S.R. and Crouch, M.L. (1987) J. Biol. Chem. 262 (25), 12202-12208.
Sengupta-Gopalan, C., Reichart, N.A., Barker, R.F., Hall, T.C. and Kemp, J.D. (1985) Proc. Natl. Acad. Sci. USA 82, 3320-3324.
sharief, P. and Li, S.S. (1982) J. Biol. Chem. 257, 14753-14759.
Simantov, R. and Snyder, S.H. (1976) Life Sci. 18, 781-788.
Stanssens, P., McKeown, Y., Friedrich, K., and Fritz, H.J. (1987) Manual EMBO Laboratory Course; 'Directed mutagensis and protein engineering held at Max Planck Institute fβüβr Biochemie, Martinsried, W-Germany,July 4-18, 1987.
Takahashi, Y., Kato, Kikuya, Hayashizaki, Y-, Wakabayashi,T., Ohtsuka, E., Matsuki, S., Ikehara, M. and Matsubara, K. (1985) Proc. Natl. Acad. Sci. U.S.A. 82, 1931-1935.
Towbin, H., Stashelin, T. and Gordon, J. (1979) Proc. Natl. Acad. Sci. (U.S.A.) 76, 4350-4354.
Velten, J., Velten, L., Hain, R. and Schell, J. (1984) EMBO J. 3, 2723-2730
Walling, L. Drews, G.N. and Goldberg, R. (1986) Proc. Natl. Acad. Sci. 83, 2123-2127.
Yang, F., Euna, V.G., McAnelly, R.D., Noberhaus, K.H., cupples, R.L., Bowman, B.H. (1985) Nucl. Acids Res. 13, 8007-8011.
yanisch-perron, C., Vieira, J. and Messing, J. (1985) Gene, 33, 103-119.
Youle, R. and Huang, A.H.C. (1981) American J. Bot. 68, 44-48.

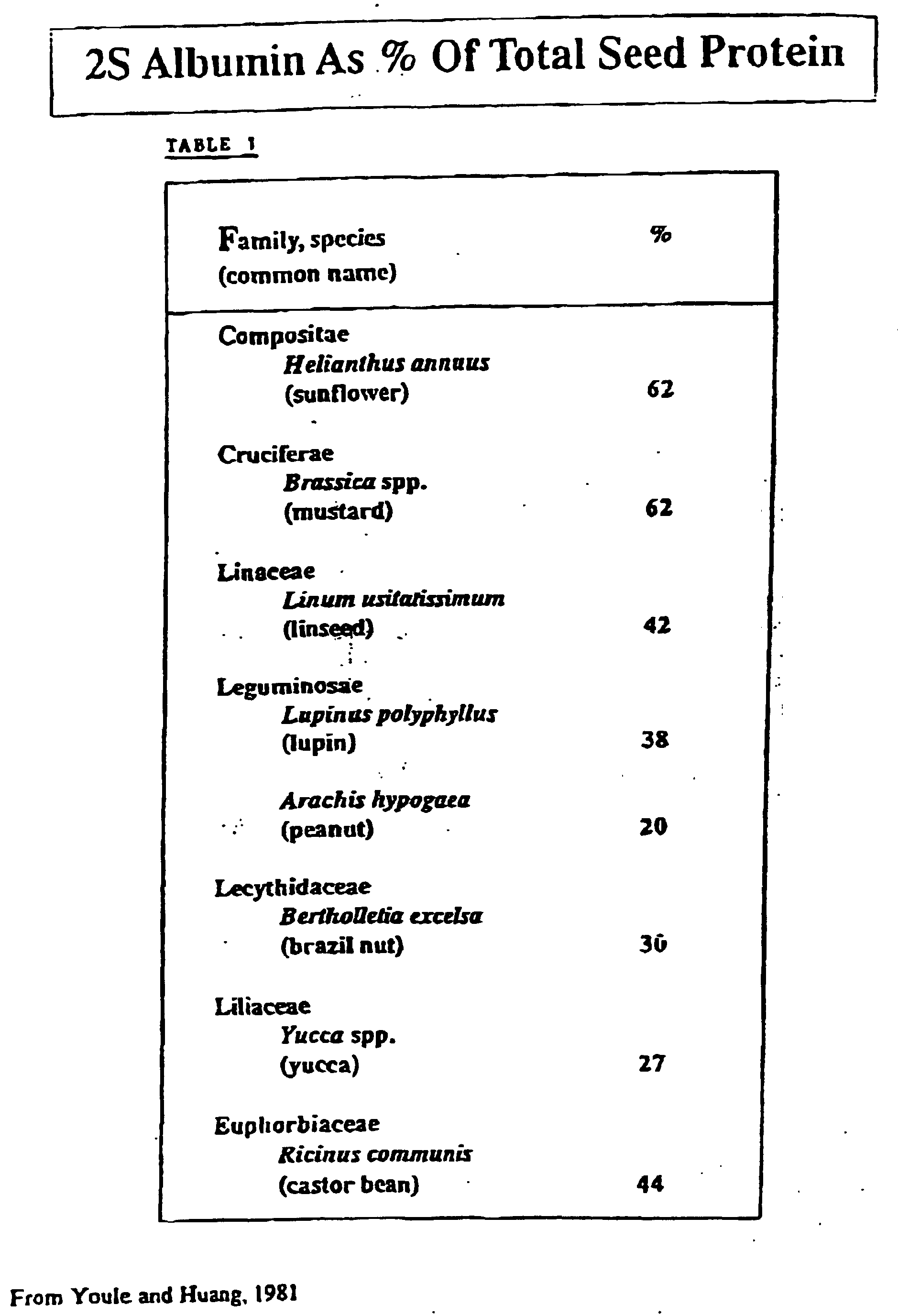

## Claims

1. A 5' flanking region of an Arabidopsis gene coding for a 2S albumin precursor, **characterized in that** said 5' flanking region comprises the nucleotide sequence of Fig 13. between position -431 and -1.

2. The 5' flanking region of claim 1 wherein said 5' flanking region is contained in plasmid pAT2S1Bg, deposited at Deutsche Sammlung von Mikroorganismen under depository number DSM 4878.

3. A chimeric gene comprising the 5' flanking region of claim 1 or claim 2.

## Patentansprüche

1. Eine 5' flankierenden Region eines Arabidopsis Gens, das für ein 2S Albumin Prekursor kodiert und daduch charakterisiert is, dass besagte 5' flankierende Region eine Nukleotidensequenz wie in Abbildung 13 gezeigt zwischen Position - 431 und -1 enthält.

2. Die 5' flankierenden Region gemäss Anspruch 1, worin besagte 5' flankierende Region enthalten ist in Plasmid pAT2S1Bg, hinterlegt in der Deutschen Sammlung von Mikroorganismen unter Hinterlegungsnummer DSM4878.

3. Ein chimäres Gen das eine 5' flankierende Region gemäss Anspruch 1 oder Anspruch 2 enthält.

## Revendications

1. Une région flanquante 5' d'un gène d'Arabidopsis codant pour un précurseur de l'albumine 2S **caractérisée en ce que** ladite région comprend une séquence de nucléotides de la figure 13 entres les positions -431 et -1.

2. La région flanquante 5' selon la revendication 1 dans laquelle ladite région flanquante 5' est contenu dans le plasmide pAT2S1Bg déposé au Deutsche Sammlung von Mikroorganismen sous numéro de dépôt DSM 4878.

3. Un gène chimère comprenant la région flanquante 5' selon la revendication 1 ou la revendication 2.
